# EUROPEAN PATENT SPECIFICATION

(11) **EP 4 467 211 B1**
(45) Date of publication and mention of the grant of the patent: **18.11.2026**
(21) Application number: 23876427.8
(22) Date of filing: 08.09.2023
(51) Int. Cl.: A63B 71/06, A61B 5/024, A61B 5/145, A61B 5/00, G06N 7/01, G16H 20/30

(54) **FITNESS AGE ESTIMATION METHOD AND ELECTRONIC DEVICE**
FITNESSALTERSCHÄTZVERFAHREN UND ELEKTRONISCHE VORRICHTUNG
PROCÉDÉ D'ESTIMATION D'ÂGE PHYSIQUE ET DISPOSITIF ÉLECTRONIQUE

(30) Priority: 14.10.2022 CN 202211260641
(43) Date of publication of application: 27.11.2024
(73) Proprietor: Honor Device Co., Ltd., Shenzhen, Guangdong 518040 (CN)
(72) Inventor: CAO, Yao, Shenzhen, Guangdong 518040 (CN); UNG, ChinYih, Shenzhen, Guangdong 518040 (CN); ZHANG, Cheng, Shenzhen, Guangdong 518040 (CN); ZHAN, Jianhua, Shenzhen, Guangdong 518040 (CN)
(74) Representative: Beder, Jens
(86) International application number: PCT/CN2023/117818
(87) International publication number: WO 2024/078222

(56) References cited:
- EP-B1- 3 046 462
- CN-A- 111 260 186
- CN-A- 112 138 361
- CN-A- 112 582 067
- CN-A- 112 967 780
- CN-A- 112 967 802
- US-A1- 2005 228 237
- US-A1- 2022 314 074
- US-B2- 9 852 266
- STORK MILAN ET AL: "CARDIOPULMONARY EXERCISE TESTING FOR VO 2 MAX DETERMINING IN SUBJECTS OF DIFFERENT PHYSICAL ACTIVITY", 1 January 2016 (2016-01-01), pages 91 - 101, XP093266065, Retrieved from the Internet <URL:https://ojs.cvut.cz/ojs/index.php/CTJ/article/view/4210/4064>

## Description

### TECHNICAL FIELD

This application relates to the technical field of terminals, and in particular, to a fitness age estimation method, an electronic device and a computer-readable storage medium.

### BACKGROUND

Studies have shown that a user with good living habits has a fitness age (that is, a physiological age) much lower than the chronological age. The fitness age is relative to the chronological age. The chronological age is a length of time since birth. The fitness age represents vitality of an individual. For example, some people have a chronological age of 50 years old, but have a fitness age of only about 40 years old. Therefore, these people are healthy and viable. In contrast, some other people have a chronological age of 40 years, but have a fitness age of 50 years old. Therefore, these people usually "look older than they really are". In other words, determining the fitness age allows for a person to be more accurately aware of his or her vitality.

In the related art, although the fitness age may be measured through instruments, most users cannot access such instruments in daily life, making it difficult to obtain fitness ages of the users.

With regard to the prior art, reference is made to Stork, M., et al.: "Cardiopulmonary exercise testing for VO2 max determining in subjects of different physical activity", Lékař a tech-nika-Clinician and Technology 46, pages 91-101, 1 January 2016. Further reference is made to the patent applications EP 3 046 462 B1, US 2022/314074 A1 and US 9 852 266 B2.

### SUMMARY

To solve the problem of estimating a fitness age based on a maximal oxygen uptake during exercise, embodiments of this application provides a fitness age estimation method, an electronic device and a computer-readable storage medium according to the enclosed independent claims. This fitness age estimation method can help to routinely obtain a fitness age, and to reduce the difficulty in obtaining the fitness age.

The invention is defined by the appended claims.

### BRIEF DESCRIPTION OF DRAWINGS

FIG. 1 is a first schematic diagram of interfaces of a watch according to an embodiment of this application;
FIG. 2 is a second schematic diagram of an interface of a watch according to an embodiment of this application;
FIG. 3 is a third schematic diagram of interfaces of a watch according to an embodiment of this application;
FIG. 4 is a schematic diagram of interfaces of a mobile phone according to an embodiment of this application;
FIG. 5 is a diagram of a hardware structure of a mobile phone according to an embodiment of this application;
FIG. 6 is a brief flowchart of calculating a maximal oxygen uptake according to an embodiment of this application;
FIG. 7 is a brief flowchart of correcting a maximal oxygen uptake according to an embodiment of this application;
FIG. 8 is a brief flowchart of estimating a fitness age according to an embodiment of this application;
FIG. 9 is a first brief flowchart of correcting a fitness age according to an embodiment of this application;
FIG. 10 is a second brief flowchart of correcting a fitness age according to an embodiment of this application;
FIG. 11 is a third brief flowchart of correcting a fitness age according to an embodiment of this application;
FIG. 12 is a fourth brief flowchart of correcting a fitness age according to an embodiment of this application;
FIG. 13 is a flowchart of a fitness age estimation method according to an embodiment of this application; and
FIG. 14 is a diagram of a structure of a chip system according to an embodiment of this application.

### DESCRIPTION OF EMBODIMENTS

An embodiment of this application provides a fitness age estimation method. The method may be applied to a wearable electronic device such as a watch, smart glasses, an earphone, or the like. The following mainly describes the solution of this application by using an example in which the electronic device is a watch. By using the method in embodiments of this application, the electronic device may calculate a fitness age of a user, and provide the fitness age for the user. In a specific implementation, the electronic device may display the fitness age, to provide the fitness age for the user.

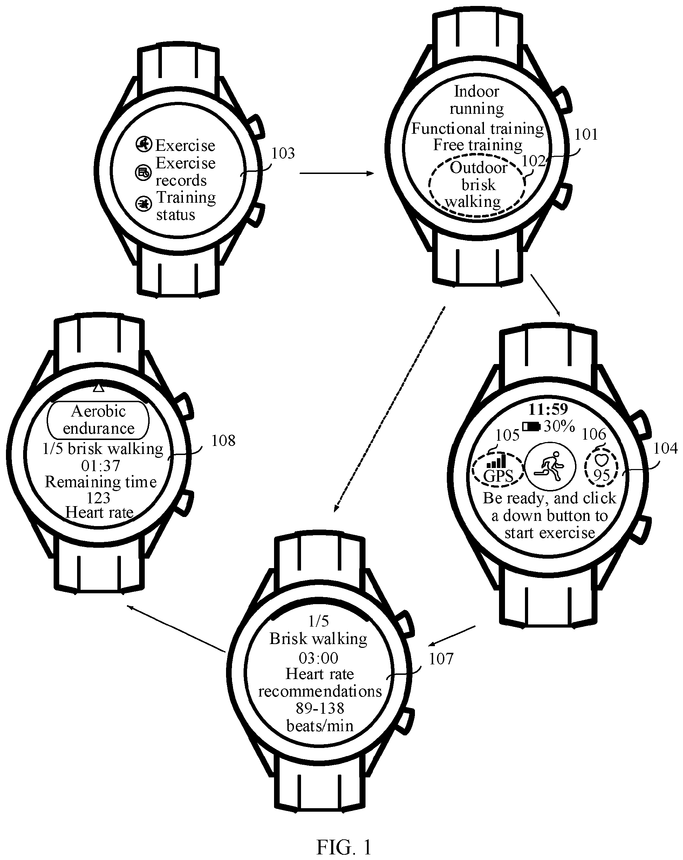

The watch may provide a target option (or may be referred to as a first control) for a specific exercise for testing a maximal oxygen uptake. For example, the specific exercise includes outdoor brisk walking, and in this case, the watch may provide an outdoor brisk walking option 102 in an interface 101 (or may be referred to as a first interface) shown in FIG. 1. That is, the target option is the outdoor brisk walking option 102. Certainly, during actual implementation, a preset exercise may alternatively be another type of exercise such as outdoor running, hill climbing, or the like.

In a specific implementation, the watch may display the interface 103 shown in FIG. 1. The interface 103 includes options such as "exercise", "exercise records", "training status", and the like. The watch may detect an operation on the "exercise" option by the user. In response to a selection operation on the "exercise" option by the user, the watch may display an interface including the target option (for example, the outdoor brisk walking option 102), thereby providing the target option. For example, the interface including the target option (for example, the outdoor brisk walking option 102) is the interface 101, where the interface 101 includes the outdoor brisk walking option 102, and further includes options such as "indoor running", "free training", and the like.

The watch may detect the operation on the target option by the user. In response to detecting the selection operation (or may be referred to as a first operation) on the target option by the user, the watch may guide the user to complete a test exercise for testing the maximal oxygen uptake.

In some embodiments, the watch needs to use positioning to calculate pace during exercise for subsequent calculation on the fitness age. In this embodiment, after detecting the selection operation on the target option by the user, the watch may display a positioning prompt to prompt whether the positioning is successful or not. For example, the watch may display an interface 104 shown in FIG. 1 after detecting the selection operation by the user on the outdoor brisk walking option 102 (where the outdoor brisk walking option 102 is the target option) in the interface 101 shown in FIG. 1. The interface 104 includes a positioning prompt 105. A global positioning system (Global Positioning System, GPS) in the positioning prompt 105 has signal fullness. This may prompt successful GPS positioning.

In some embodiments, the watch needs to calculate the fitness age based on a heart rate during exercise. In this embodiment, the watch may display a heart rate prompt after detecting the selection operation on the target option by the user, where the heart rate prompt may further include a heart rate recommendation prompt and a real-time heart rate prompt. In this way, the user may be guided to control the heart rate during exercise.

For example, the watch may display an interface 104 shown in FIG. 1 after detecting the selection operation by the user on the outdoor brisk walking option 102 (where the outdoor brisk walking option 102 is the target option) in the interface 101 shown in FIG. 1. The interface 104 includes a real-time heart rate prompt 106, where the real-time heart rate prompt 106 display a resting heart rate of the user before exercise of 95 beats per minute.

As shown in FIG. 1, the interface 104 may further include an operation prompt for starting exercise, for example, "Be ready, and click a down button to start exercise." In this way, the user is guided to start the test exercise.

In another example, the watch may display an interface 107 shown in FIG. 1 after detecting a user's operation of triggering to start the test exercise, for example, detecting a user's operation of clicking the down button in the interface 104 shown in FIG. 1. The interface 107 includes the heart rate recommendation prompt, for example "A heart rate recommendation is 89-138 beats/minute", that is, a heart rate recommendation is 89-138 beats per minute.

Certainly, the operation of triggering to start the test exercise may alternatively be the selection operation on the target option by the user. That is, after detecting the selection operation on the target option by the user, the watch may directly start the test exercise, for example, display the interface 107.

In some embodiments, after detecting the user's operation of triggering to start the test exercise, the watch may receive and display a remaining duration prompt, to prompt remaining duration of the test exercise. For example, the watch may display the interface 107 shown in FIG. 1 after detecting the user's operation of clicking the down button in the interface 104 shown in FIG. 1. The interface 107 includes the remaining duration prompt, for example, remaining duration is 3 minutes. Alternatively, as time goes by, the watch may display an interface 108 shown in FIG. 1, where the interface 108 includes the remaining duration prompt, for example, remaining duration is 1 minute and 37 seconds.

In some embodiments, the test exercise includes a plurality of test stages, and the test stages are used to test for different fitness situations, such as testing for endurance, explosion, and the like. In this embodiment, the watch may display a test stage prompt after detecting the user's operation of triggering to start the test exercise. For example, the watch may display the interface 107 shown in FIG. 1 after detecting the user's operation of clicking the down button in the interface 104 shown in FIG. 1. The interface 107 includes the test stage prompt, for example, prompting that the test stage is "1/5 brisk walking", that is, the current test stage is the first stage in five stages of brisk walking. Alternatively, as time goes by, the watch may display the interface 108 shown in FIG. 1. The interface 108 includes the test stage prompt, for example, prompting that the test stage is "1/5 brisk walking", and prompting that the stage is mainly for an "aerobic endurance" test.

It is to be understood that although various prompts (or may be referred to as test guide information) for guiding the user to complete the test exercise have been described separately, during actual implementation, the prompts may be used in combination. It is to be noted that in the embodiment in which the plurality of test stages are divided, the heart rate recommendation prompt is a heart rate recommendation at a corresponding test stage, the real-time heart rate is a real-time heart rate detected at the corresponding test stage, and the remaining duration is remaining duration of the corresponding test stage. For example, the watch may display the interface 107 shown in FIG. 1. The test stage prompt in the interface 107 is "1/5 brisk walking". The heart rate recommendation prompt "A recommended heart rate is in a range of 89 to 138 beats/minute" in the interface 107 indicates that a recommended heart rate for the "1/5 brisk walking" stage is in a range of 89 to 138 beats per minute. The remaining duration prompt "03:00" in the interface 107 indicates that the remaining duration of the "1/5 brisk walking" stage is 3 minutes.

After the watch detects that the user completes the test exercise, for example, five test stage for brisk walking end, or the watch detects a user's operation of completing the test exercise, the watch may calculate the maximal oxygen uptake, that is, VO2max based on exercise data such as an exercise heart rate, an exercise speed, and the like detected during the test exercise. Then, the fitness age is estimated based on VO2max.

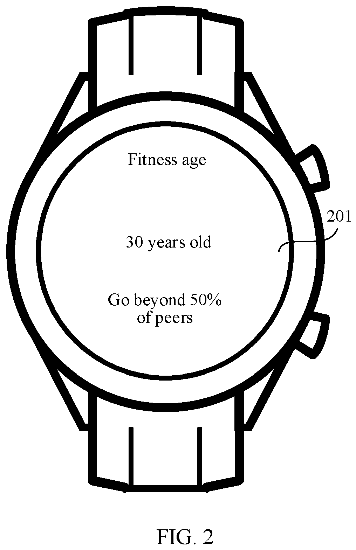

**In** some embodiments, after estimation on the fitness age is completed, the watch may display a currently estimated fitness age. For example, after the fitness age is estimated, the watch may display an interface 201 shown in FIG. 2, where the interface 201 includes a value of the fitness age, for example, 30 years old. For ease of description, an interface displaying the currently estimated fitness age is referred to as a second interface.

After the fitness age is estimated, the watch may further display information indicating a level of the fitness age. For example, the interface 201 further includes information of "Go beyond 50% of peers" for indicating that the fitness age of the user is superior to 50% of peers.

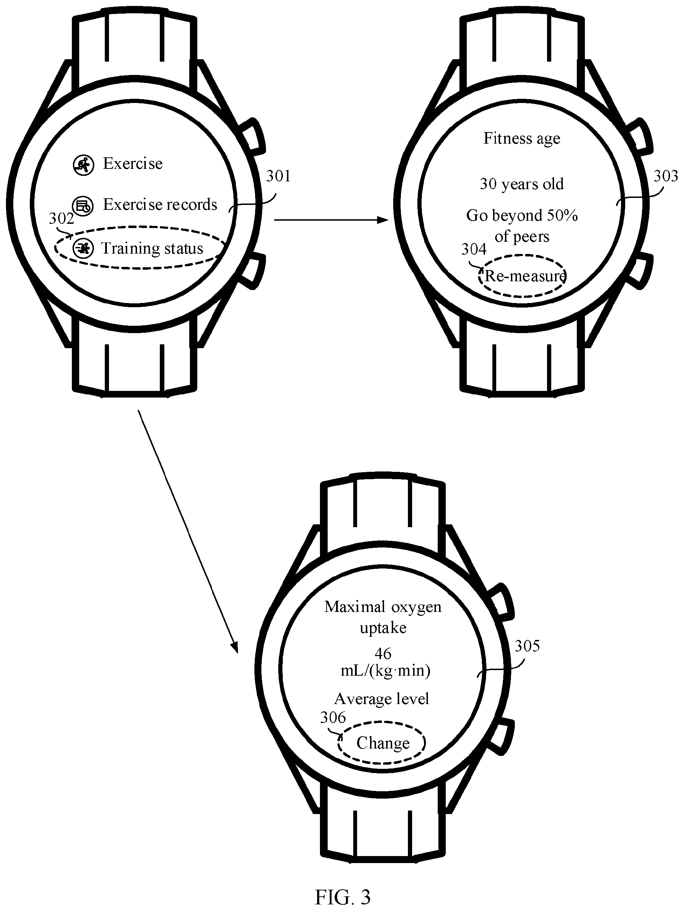

Further, the watch may further provide a function of viewing historically estimated fitness ages. In this way, after exiting an interface displaying the fitness age, for example, exiting the interface 201, the watch may also provide historical data for viewing by the user. For example, the watch may display an interface 301 shown in FIG. 3, where the interface 301 includes a training status option 302. After detecting a selection operation by the user on the training status option 302, the watch may display historical training data such as a training load, a maximal oxygen uptake, and a fitness age. The user may perform a slide operation to trigger the watch to slide to display various training data. For example, after the user slides to the fitness age, the interface 303 shown in FIG. 3 may be displayed, where the interface 303 includes the last estimated fitness age. For ease of description, an interface displaying the historically estimated fitness age is referred to as a third interface.

As shown in FIG. 3, the interface 303 further includes a re-measurement option 304 for triggering re-estimation on the fitness age. After detecting a selection operation by the user on the re-measurement option 304, the watch may re-guide the user to complete the test exercise. For example, the user may be guided to complete the test exercise according to processes of the interface 104, the interface 107, and the interface 108 shown in FIG. 1. Details are not described herein. In addition, the watch may display an updated fitness age after the test exercise is completed. For ease of description, a control (for example, the re-measurement option 304) for triggering the watch to re-estimate the fitness age is referred to as a second control, and an operation of triggering the watch to re-estimate the fitness age is referred to as a second operation.

In some embodiments, after the fitness age is estimated, the watch may transmit the currently estimated fitness age to another electronic device such as a mobile phone, a tablet, or the like. Descriptions are provided below mainly by using an example in which the another electronic device is a mobile phone. After receiving the fitness age, the mobile phone may perform statistics analysis on the fitness age of the user.

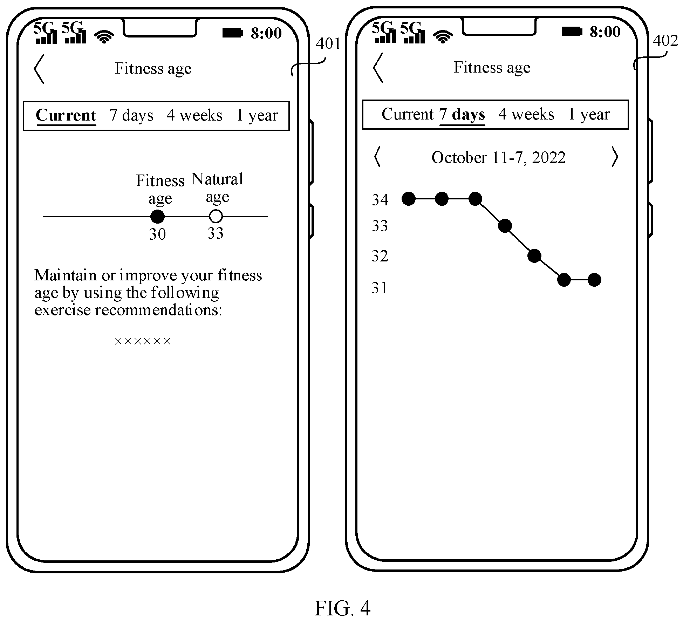

For example, a sport and health application (Application, APP) is installed in the mobile phone, and the sport and health APP in the mobile phone may perform statistics analysis on the fitness age of the user after receiving the fitness age. Subsequently, the mobile phone may display an analysis result for the user after detecting that the user views the fitness age recorded in the sport and health APP. For example, the mobile phone may display an interface 401 shown in FIG. 4, where the interface 401 includes viewing options for a plurality of time intervals, for example, four viewing options for current time, 7 days, 4 weeks, and 1 year. The mobile phone may display a corresponding analysis result after a viewing option is selected. For example, after a current-time viewing option is selected, the mobile phone may display a result of comparison between a fitness age and a chronological age, as shown in the interface 401. In another example, after a 7-day viewing option is selected, the phone may display a curve of changes in the fitness age of the user in over 7 days, as shown in the interface 402.

By using the fitness age estimation method provided in embodiments of this application, after the test exercise is completed, the fitness age may be estimated based on the exercise data such as the exercise heart rate, the exercise speed, and the like detected during the test exercise. In embodiments of this application, the watch may first calculate a maximal oxygen uptake during the test exercise based on the exercise data such as the exercise heart rate, the exercise speed, and the like, and then calculate a fitness age based on the maximal oxygen uptake. In this way, the fitness age of the user may be estimated after daily exercise is completed. This can routinely obtain a fitness age, and reduce the difficulty in obtaining the fitness age.

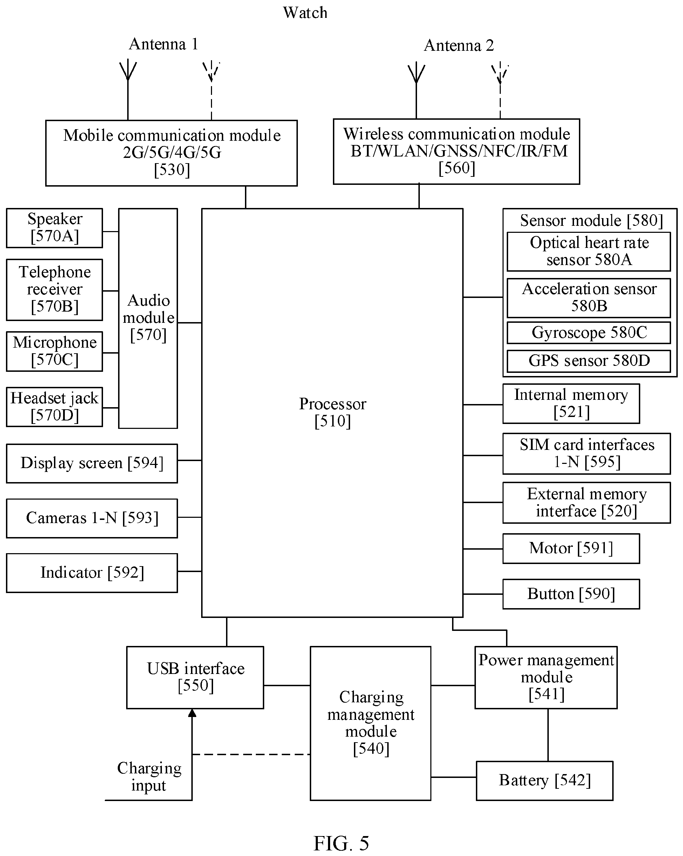

As shown in FIG. 5, the watch is used as an example, the electronic device provided in embodiments of this application may include a processor 510, an external memory interface 520, an internal memory 521, a universal serial bus (universal serial bus, USB) interface 530, a charging management module 540, a power management module 541, a battery 542, an antenna 1, an antenna 2, a mobile communication module 550, a wireless communication module 560, and an audio module 570, a speaker 570A, a telephone receiver 570B, a microphone 570C, a headset jack 570D, a sensor module 580, a button 590, a motor 591, an indicator 592, a camera 593, a display screen 594, a subscriber identity module (subscriber identity module, SIM) card interface 595, and the like.

The sensor module 580 includes, but is not limited to, an optical heart rate sensor 580A, an accelerometer (Accelerometer, ACC) sensor 580B, a gyroscope (Gyroscope, GYRO) 580C, and a GPS sensor 580D.

It may be understood that the structure illustrated in this embodiment does not constitute a specific limitation on the watch. In some other embodiments, the watch may include more or fewer components than those shown in the figure, or some components may be combined, or some components may be split, or components are arranged in different manners. The components in the figure may be implemented by hardware, software, or a combination of software and hardware.

The processor 510 may include one or more processing units. For example, the processor 510 may include an application processor (application processor, AP), a modem processor, a graphics processing unit (graphics processing unit, GPU), an image signal processor (image signal processor, ISP), a controller, a memory, a video codec, a digital signal processor (digital signal processor, DSP), a baseband processor, and/or a neural-network processing unit (neural-network processing unit, NPU), and the like. Different processing units may be separate devices, or may be integrated into one or more processors. In some embodiments, the processor 510 may perform the following processing: calculating a maximal oxygen uptake during the test exercise based on data such as a heart rate, a speed, and the like, and further calculating a fitness age based on the maximal oxygen uptake.

The charging management module 540 is configured to receive a charging input from a charger. The charger may be a wireless charger or a wired charger. In some embodiments of wired charging, the charging management module 540 may receive a charging input of a wired charger by using the USB interface 530. In some embodiments of wireless charging, the charging management module 540 may receive a wireless charging input by using a wireless charging coil of the mobile phone 500. The charging management module 540 may further supply power to the watch through the power management module 541 while charging the battery 542.

The power management module 541 is configured to connect the battery 542, the charging management module 540, and the processor 510. The power management module 541 receives an input of the battery 542 and/or the charging management module 540, to supply power to the processor 510, the internal memory 521, an external memory, a display screen 594, the camera 593, the wireless communication module 560, and the like. The power management module 541 may be further configured to monitor parameters such as a battery capacity, battery recycling times, and a battery health status (leakage and impedance). In some other embodiments, the power management module 541 may alternatively be disposed in the processor 510. In some other embodiments, the power management module 541 and the charging management module 540 may alternatively be disposed in a same device.

A wireless communication function of the watch may be implemented by using the antenna 1, the antenna 2, the mobile communication module 550, the wireless communication module 560, the modem processor, the baseband processor, and the like.

The wireless communication module 560 may provide solutions of wireless communication applied to the watch, including a wireless local area network (wireless local area network, WLAN) (such as a wireless fidelity (wireless fidelity, Wi-Fi) network) and Bluetooth (bluetooth, BT), a global navigation satellite system (global navigation satellite system, GNSS), frequency modulation (frequency modulation, FM), near field communication (near field communication, NFC), and infrared (infrared, IR). The wireless communication module 560 may be one or more devices that integrate at least one communication processing module. The wireless communication module 560 receives an electromagnetic wave by using the antenna 2, performs frequency modulation and filtering processing on an electromagnetic wave signal, and sends a processed signal to the processor 510. The wireless communication module 560 may alternatively receive a to-be-sent signal from the processor 510, perform frequency modulation and amplification on the to-be-sent signal, and convert the signal into an electromagnetic wave for radiation by using the antenna 2.

The watch implements a display function by using the GPU, a display screen 594, the application processor, and the like. The GPU is a microprocessor for image processing, and is connected to the display screen 594 and the application processor. The GPU is configured to perform mathematical and geometric calculation, and is configured to render graphics. The processor 510 may include one or more GPUs, and the GPU executes program instructions to generate or change display information. In some embodiments, the display screen 594 may be configured to display a fitness age, and display information for guiding the user to complete a test exercise. For example, the display screen 594 of the watch may display content in interfaces in FIG. 1 to FIG. 3.

The watch may implement a photographing function by using an ISP, the camera 593, a video codec, the GPU, the display screen 594, the application processor, and the like. The ISP is configured to process data fed back by the camera 593. The camera 593 is configured to capture a static image or a video. An optical image is generated for an object by using the lens and is projected onto the photosensitive element.

The watch may include one or N cameras 593, where N is a positive integer greater than 1. For example, the camera 593 may include a depth camera, a binocular camera and/or a monocular camera.

The external memory interface 520 may be configured to connect to an external storage card such as a micro SD card, to extend a storage capability of the watch. The external storage card communicates with the processor 510 by using the external memory interface 520, to implement a data storage function, for example, storing a file such as a music or a video in the external storage card.

The internal memory 521 may be configured to store computer executable program code, where the executable program code includes instructions. The processor 510 runs the instructions stored in the internal memory 521, to perform various function applications and data processing of the watch. For example, the processor 510 may display different content on the display screen 594 in response to the user's operation of expanding the display screen 594 by executing instructions stored in the internal memory 521. The internal memory 521 may include a program storage area and a data storage area. The program storage area may store an operating system, an application required by at least one function (such as a voice playing function and an image playing function), and the like. The data storage area may store data (for example, audio data or a phone book) and the like created during use of the watch. In addition, the internal memory 521 may include a high-speed random access memory, and may also include a non-volatile memory, for example, at least one magnetic disk storage device, a flash memory device, or a universal flash storage (universal flash storage, UFS).

The watch may implement an audio function, for example, music playback or recording, by using the audio module 570, the speaker 570A, the phone receiver 570B, the microphone 570C, the headset jack 570D, the application processor, and the like.

The optical heart rate sensor 580A is currently one of the most popular sensors for heart rate detection. The optical heart rate sensor measures a heart rate and other biometrics by using Photoplethysmography (Photoplethysmography, PPG). A measurement principle of the PPG is as follows: Light is directed to the skin by a capacitive lamp, light reflected back through the skin tissue is received by a photosensitive sensor and converted into an electrical signal, the electrical signal is converted into a digital signal via the electrical signal, and a heart rate is calculated based on an absorbance of blood.

The ACC sensor 580B may be configured to detect a value of acceleration of the watch in various directions (generally three axes). The GYRO 580C may be configured to detect an angular velocity of the watch about three axes (e.g., x, y, and z axes). The GPS sensor 580D may be configured to position the watch.

In some embodiments, the watch may use the optical heart rate sensor 580A, the ACC sensor 580B, and the GYRO 580C to detect a real-time heart rate of the user during fitness age detection. The optical heart rate sensor 580A may detect a heart rate signal included and may further detect a noise signal such as ambient light. The ACC sensor 580B and the GYRO 580C may filter out the noise signal from the signal detected by the optical heart rate sensor 580A, to obtain the heart rate signal. The watch may use the ACC sensor 580B, the GYRO 580C, and the GPS sensor 580D to detect an exercise speed of the user during fitness age detection. Specific implementations of detecting the heart rate and the speed are not described herein. For details, reference may be made to descriptions in the related art.

The button 590 includes a power button, a volume button, and the like. The button 590 may be a mechanical button, or a touch-type button. The watch may receive a key-based input, and generate a key signal input related to a user setting and function control of the watch. The motor 591 can generate a vibration prompt. The motor 591 may be configured to provide a vibration prompt for an incoming call, and may be further configured to provide a touch vibration feedback. The indicator 592 may be an indicator light, and may be configured to indicate a charging state or a battery change, or may be further configured to indicate a message, a missed call, a notification, or the like. The SIM card interface 595 is configured to connect to a SIM card. The SIM card may be inserted into the SIM card interface 595 or removed from the SIM card interface 595, to come into contact with or be separated from the watch. The watch may support one or N SIM card interfaces, where N is a positive integer greater than 1.

The fitness age estimation method provided in embodiments of this application may be implemented by the watch having the foregoing hardware structure.

The watch may detect the exercise heart rate and the exercise speed when the user performs the test exercise. For example, the exercise heart rate is detected through the optical heart rate sensor 580A, the ACC sensor 580B, and the GYRO 580C, and the exercise speed is detected through the ACC sensor 580B, the GYRO 580C, and the GPS sensor 580D. After detecting the end of the test exercise or a user's operation of triggering to end the test exercise, the watch may calculate a maximal oxygen uptake VO2max during the test exercise based on the exercise heart rate and the exercise speed, and estimate a fitness age of the user based on the maximal oxygen uptake.

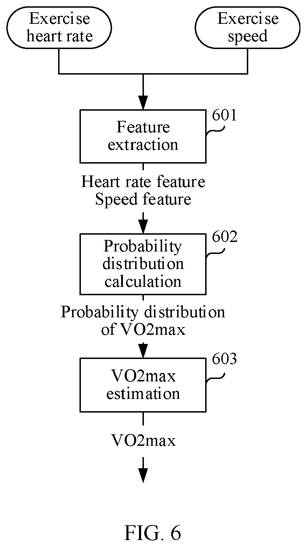

In some embodiments, referring to FIG. 6, that the watch calculates the maximal oxygen uptake based on the exercise heart rate and the exercise speed includes: performing feature extraction on the exercise heart rate and the exercise speed (feature extraction shown as 601 in FIG. 6); calculating a probability distribution of VO2max based on a heart rate feature and a speed feature that are extracted (probability distribution calculation shown as 602 in FIG. 6); and estimating VO2max based on the probability distribution of VO2max (VO2max estimation shown as 603 in FIG. 6). Descriptions are separately provided below.

Feature extraction is first described.

By using a feature engineering method, the watch extracts the heart rate feature based on a resting heart rate before the test exercise and a real-time heart rate per second during the test exercise, and extracts the speed feature based on an exercise speed per second during the test exercise. The heart rate feature includes, but is not limited to, a statistical feature related to a resting heart rate, a maximum heart rate, and heart rate time and frequency domain, and a derived feature on a timing period. The speed feature includes, but is not limited to, statistical features and derived features on an average speed, a maximum speed, and a speed timing.

Then, a probability distribution is calculated. The probability distribution includes a plurality of possible VO2max after the user completes the test exercise, and a probability for each possible VO2max.

The watch may run a maximal oxygen uptake prediction model by using the heart rate feature and the velocity feature that are extracted as inputs, to obtain a probability distribution of the maximal oxygen uptake. The maximal oxygen uptake prediction model may be a Bayesian regression analysis model, a support vector machine model, a tree model, or a neural network model. This is not specifically limited in embodiments of this application.

In a specific implementation, the watch may calculate a probability distribution *p*(VO2max | HRn, w) by using the Bayesian regression analysis model shown in Formula (1) below. $p \left(\left.VO 2max\right|HRn,w\right) = \frac{p \left(\left.HRn, w\right|VO 2max\right) p \left(VO 2max\right)}{p \left(HRn, w\right)}$ Formula (1)

HRn represents a normalized heart rate, and w represents an exercise load.

It is to be noted that, in some implementations, a previously calculated probability distribution is required in the probability distribution calculation. Therefore, each time the probability distribution calculation is completed, the calculated probability distribution needs to be stored for next probability distribution calculation. To reduce data storage in the watch, after obtaining a probability distribution each time, the watch may transmit the probability distribution to another electronic device such as a mobile phone, a tablet, or the like communicatively connected to the watch. Then, the probability distribution is stored by the another electronic device. Subsequently, when probability distribution calculation needs to be completed, the watch may obtain a historical probability distribution from the another electronic device.

Through the probability distribution calculation, the watch can obtain a probability that VO2max of the user is equal to each VO2max value in a preset interval. For example, the preset interval is 20-90 mL/(kg min), and probabilities that VO2max is equal to 71 VO2max values ranging from 20 to 90 may be obtained. For example, a calculated probability distribution result for VO2max is [k1, k2, k3, ..., k71], indicating that probabilities that VO2max is equal to 20 mL/(kg min), 21 mL/(kg min), 22 mL/(kg min), ..., and 90 mL/(kg min) are sequentially k1, k2, k3, ..., and k71. k1, k2, k3, ..., and k71 are all in a range of 0 to 1, and k1+k2+k3, ..., k71=1.

Then, VO2max is estimated.

After a probability distribution of VO2max is obtained, the watch may estimate VO2max. In some embodiments, the watch may obtain, from the probability distribution of VO2max, an oxygen uptake with the largest probability as VO2max. For example, if k25 of k1 to k71 has the largest probability, an oxygen uptake corresponding to k25 is 44, and it may be determined that VO2max is 44. In some other embodiments, the watch may perform weighted summation on an oxygen uptake and a probability corresponding to the oxygen uptake, to obtain VO2max. For example, VO2max=20*k1+21*k2+22*k3+90*k71.

VO2max may be obtained through the process shown in FIG. 6 for subsequent calculation on the fitness age.

Certainly, during actual implementation, a person skilled in the art may also calculate VO2max by using relevant existing technologies, which is not limited to the manner of calculating VO2max shown in FIG. 6.

In some scenarios, the exercise heart rate and the exercise speed captured by the sensor may be inaccurate, and user's exercise during the test exercise may not meet a test condition, for example, there may be a deviation in calculated VO2max due to factors such as insufficient exercise time, an exercise heart rate not controlled within the range of the recommended heart rate, and the like.

Based on this, in some embodiments, after VO2max is calculated, the watch may correct VO2max based on a target population to which the user belongs, to improve rationality of VO2max. The population may be classified into three classes, namely, lean, fat, and normal according to a body mass index (Body Mass Index, BMI). Alternatively, a plurality of classes of populations corresponding to a plurality of chronological age ranges may be classified, for example, the populations may be classified into three classes, namely, a population aged 20-30 years old, a population aged 30-40 years old, and a population aged 40-50 years old. Alternatively, the population may be classified according to a body fat percentage. Generally, a higher body fat percentage indicates a higher fat content and a poor physical condition; or a lower body fat percentage indicates a lower fat content and a better physical condition. Based on this, the populations may be classified into populations with different physical conditions according to body fat percentages. Further, the population can be classified into males and females according to genders, for example, the population is classified into leaner, fatter, and normal females, and leaner, fatter, and normal males.

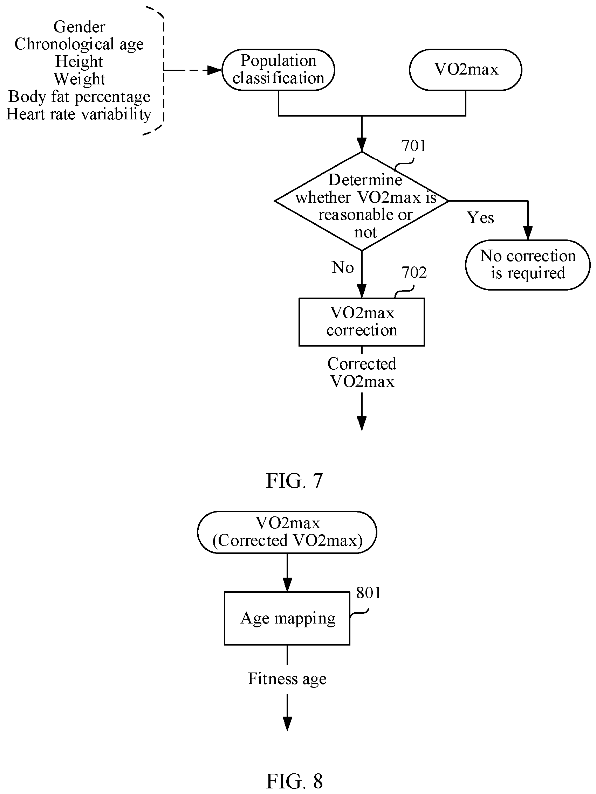

Referring to FIG. 7, VO2max correction include: determining whether VO2max is proper or not based on a proper range of VO2max (or may be referred to as a second oxygen uptake range) of the target population (that is, a VO2max proper or not determining process shown as 701 in FIG. 7); and if VO2max is not proper, correcting VO2max to obtain corrected VO2max (that is, a VO2max correction process shown as 702 in FIG. 7). Descriptions are separately provided below.

First, whether VO2max is proper or not is determined.

Each population classification has its proper VO2max range. For example, a proper range of VO2max for males aged 20-30 years old is 30-50 mL/(kg min). In another example, a proper range of VO2max is generally higher for a population with a low body fat percentage. The watch may compare VO2max calculated to a proper range of VO2max of the target population. If VO2max calculated is in the proper range of VO2max of the target population, it is determined that VO2max calculated is proper. Conversely, if VO2max calculated is not in the proper range of VO2max of the target population, it is determined that VO2max calculated is improper. In this case, the watch needs to perform VO2max correction.

Then, VO2max is corrected.

The watch may correct VO2max to a preset VO2max. The preset VO2max may be a mean value of VO2max of the target population, or may be a median of VO2max of the target population, or may be any value such as an intermediate value in the proper range of VO2max of the target population. In this way, the deviation of VO2max may be reduced.

Embodiments of this application exemplarily describe a process of obtaining the target population by the watch. Still refer to FIG. 7, based on target parameters such as a gender, a chronological age, a height, a weight, a body fat percentage, heart rate variability (Heart Rate Variability, HRV), the watch may determine the target population to which the user belongs. For example, the watch may calculate the BMI of the user based on the height and the weight, thereby determining the target population to which the user belongs. In another example, based on an age range to which the chronological age belongs, the watch may determine the target population to which the user belongs. The target parameters may be obtained by the watch receiving an input by the user, or the target parameters may be obtained by the watch from another electronic device.

Certainly, in some other embodiments, the target population may be determined by the another electronic device based on the target parameters, and then transmitted to the watch.

Referring to FIG. 8, after VO2max (or corrected VO2max, either referred to as target VO2max hereinafter), the watch may query a mapping relationship between a fitness age range and a range of VO2max corresponding to the user's gender, to determine a fitness age matching the target VO2max (that is, an age mapping process shown as 801 in FIG. 8).

For example, the mapping relationship between the fitness age and VO2max is shown in Table 1 below.

**Table 1**

| Age bracket | Male | Female |
|---|---|---|
| 18-25 years old | 43-45 mL/(kg min) | 37-42 mL/(kg min) |
| 26-35 years old | 39-42 mL/(kg min) | |
| 36-45 years old | 36-38 mL/(kg min) | 33-36 mL/(kg min) |
| 46-55 years old | 32-35 mL/(kg min) | 29-31 mL/(kg min) |
| 56-65 years old | 30-32 mL/(kg min) | 26-28 mL/(kg min) |
| 65 years old or above | 26-28 mL/(kg min) | 23-25 mL/(kg min) |

As can be reflected in Table 1, a range of VO2max of males with a fitness age of 18-25 years old is 43-45mL/(kg·min), a range of VO2max of males with a fitness age of 26-35 years old is 39-42mL/(kg·min), ..., and a range of VO2max of males with a fitness age of 65 years old or above is 26-28mL/(kg·min). In addition, as can be reflected in Table 1, a range of VO2max of females with a fitness age of 18-35 years old is 37-42mL/(kg·min); a range of VO2max of females with a fitness age of 36-45 years old is 33-36mL/(kg·min), ..., and a range of VO2max of females with a fitness age of 65 years old or above is 23-25mL/(kg·min).

Further, to determine the fitness age more accurately, the mapping relationship between the fitness age range and the range of VO2max may be refined, to obtain a mapping relationship between each fitness age and VO2max. In a specific implementation, the range of VO2max may be divided evenly based on a plurality of fitness ages included in the corresponding fitness age range. The males aged 18-25 years old in Table 1 is used as an example, a range of VO2max of the males is 43-45 mL/(kg-min), a difference 2 between an upper limit and a lower limit of 43-45 mL/(kg-min) may be divided by a value of the fitness ages included in a range of 18-25 years old (that is, a total of 8 for 18, 19, 20, 21, 22, 23, 24, and 25) minus 1, to obtain a result of about 0.285. In this way, it can be incremented at intervals of 0.285 (or 0.28 alternating with 0.29), and a mapping relationship between each fitness age and VO2max of males aged 18-25 years old is shown in Table 2 below.

**Table 2**

| Males aged 18-25 | 43-45 mL/(kg min) |
|---|---|
| 18 years old | 45mL/(kg·min) |
| 19 years old | 44.71 mL/(kg·min) |
| 20 years old | 44.43 mL/(kg min) |
| 21 years old | 44.14 mL/(kg min) |
| 22 years old | 43.86 mL/(kg min) |
| 23 years old | 43.57 mL/(kg min) |
| 24 years old | 43.29 mL/(kg min) |
| 25 years old | 43 mL/(kg min) |

Similarly, mapping relationships between all fitness age ranges and ranges of VO2max in Table 1 are refined, so that a mapping relationship between a fitness age and VO2max can be obtained as shown in Table 3 below. A unit of age (that is, years old) and a unit of VO2max, that is, mL/(kg min) are omitted, to simplify the table.

**Table 3**

| | | | | | | | | | | |
|---|---|---|---|---|---|---|---|---|---|---|
| Age | 18 | 19 | 20 | 21 | 22 | 23 | 24 | 25 | | |
| Female VO2max | 42 | 41.71 | 41.41 | 41.12 | 40.82 | 40.53 | 40.23 | 39.94 | | |
| Male | 45 | 44.71 | 44.43 | 44.14 | 43.86 | 43.57 | 43.29 | 43 | | |
| Age | 18 | 19 | 20 | 21 | 22 | 23 | 24 | 25 | | |
| VO2max | | | | | | | | | | |
| Age | 26 | 27 | 28 | 29 | 30 | 31 | 32 | 33 | 34 | 35 |
| Female VO2max | 39.64 | 39.35 | 39.05 | 38.76 | 38.46 | 38.17 | 37.87 | 37.58 | 37.28 | 37 |
| Male VO2max | 42 | 41.67 | 41.33 | 41.00 | 40.67 | 40.33 | 40.00 | 39.67 | 39.33 | 39 |
| Age | 36 | 37 | 38 | 39 | 40 | 41 | 42 | 43 | 44 | 45 |
| Female VO2max | 36 | 35.67 | 35.33 | 35.00 | 34.67 | 34.33 | 34.00 | 33.67 | 33.33 | 33 |
| Male VO2max | 38 | 37.78 | 37.56 | 37.33 | 37.11 | 36.89 | 36.67 | 36.44 | 36.22 | 36 |
| Age | 46 | 47 | 48 | 49 | 50 | 51 | 52 | 53 | 54 | 55 |
| Female VO2max | 31 | 30.78 | 30.56 | 30.33 | 30.11 | 29.89 | 29.67 | 29.44 | 29.22 | 29 |
| Male VO2max | 35 | 34.67 | 34.33 | 34.00 | 33.67 | 33.33 | 33.00 | 32.67 | 32.33 | 32 |
| Age | 56 | 57 | 58 | 59 | 60 | 61 | 62 | 63 | 64 | 65 |
| Female VO2max | 28 | 27.78 | 27.56 | 27.33 | 27.11 | 26.89 | 26.67 | 26.44 | 26.22 | 26 |
| Male VO2max | 32 | 31.78 | 31.56 | 31.33 | 31.11 | 30.89 | 30.67 | 30.44 | 30.22 | 30 |
| Age | 66 | 67 | 69 | 71 | 73 | 75 | 77 | 79 | 81 | 83 |
| Female VO2max | 25 | 24.78 | 24.56 | 24.33 | 24.11 | 23.89 | 23.67 | 23.44 | 23.22 | 23 |
| Male VO2max | 28 | 27.78 | 27.56 | 27.33 | 27.11 | 26.89 | 26.67 | 26.44 | 26.22 | 26 |

Then, after obtaining the target VO2max, the watch may query the target VO2max from the refined mapping relationship corresponding to the gender of the user, and determine a fitness age having the mapping relationship with VO2max having the smallest difference from the target VO2max as a fitness age corresponding to the target VO2max. For example, if the target VO2max is 43.6 mL/(kg min), and the gender of the user is male, Table 3 is queried, it may be determined that VO2max with the smallest difference from 43.6 mL/(kg min) in the refined mapping relationship corresponding to male is 43.57, and a fitness age corresponding to 43.6 mL/(kg min) is 23 years old having a mapping relationship with 43.57.

With the foregoing scheme, the watch can determine the fitness age corresponding to the target VO2max only by querying the mapping relationship.

In addition, the user may be a professional user, such as a professional athlete, who is very clear of target VO2max of the user. Based on this, in some embodiments, the watch may provide a preset entry for inputting the target VO2max. After detecting a user's operation of inputting the target VO2max through the preset entry, the watch may directly obtain the fitness age through mapping based on the target VO2max inputted by the user.

For example, after detecting a selection operation by the user on the training status option 302 in the interface 301 of FIG. 3, the watch may display historical training data, such as a training load, VO2max, and a fitness age. The user may perform a slide operation to trigger the watch to slide to display various training data. For example, after the user slides to a VO2max option, an interface 305 may be displayed. A change option in the interface 305 is a preset entry. After detecting a user's operation of selecting the change option 306 and inputting corresponding target VO2max, the watch may directly obtain the fitness age through mapping based on the target VO2max inputted by the user.

However, for a non-professional user, before the user first completes the test exercise, a chronological age of the user may be defaulted as the fitness age and displayed in the watch.

After obtaining the fitness age, the watch may correct the fitness age, to make the fitness age more proper.

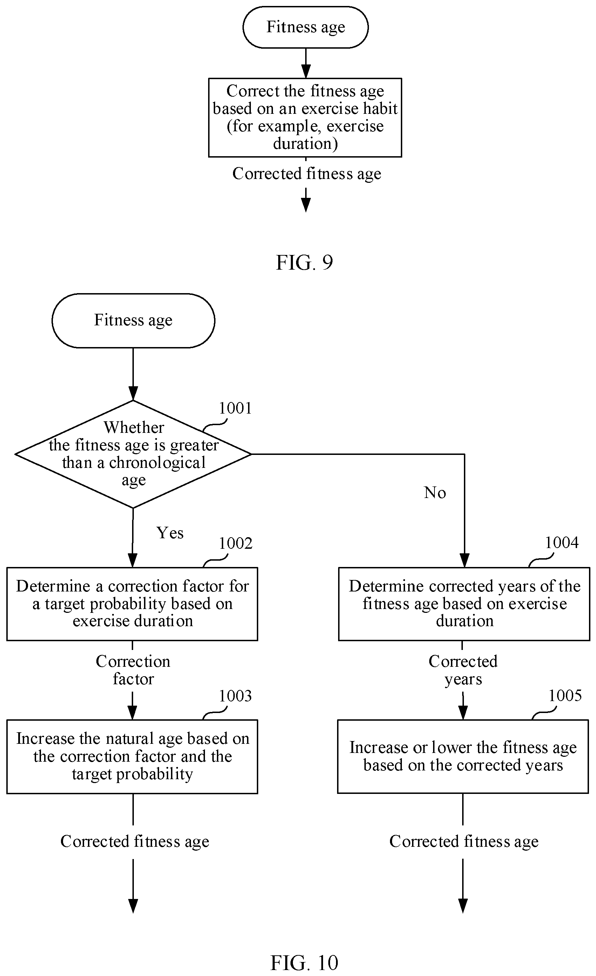

In some embodiments, referring to FIG. 9, the watch may correct the fitness age based on user's exercise habits, to obtain a corrected fitness age. The exercise habits may be measured by using parameters such as exercise frequency, exercise duration, and the like, where higher exercise frequency indicates better exercise habits, and longer exercise duration indicates better exercise habits. Descriptions are provided below mainly by using exercise duration as an example. In this embodiment, the fitness age is corrected, so that a fitness age obtained through correction when an exercise habit becomes better may be lower than a fitness age obtained through correction when an exercise habit becomes better.

In some cases, if the user likes sports in daily life, the calculated fitness age may be less than the chronological age, it indicates that the physical fitness of the user is good. For example, the fitness age is 33 years old, and the chronological age is 35 years old. In some cases, if the user is under high stress and has less daily exercise, the calculated fitness age may be greater than the chronological age, it indicates that the physical fitness of the user is poor. For example, the fitness age is 33 years old, and the chronological age is 30 years.

Further, referring to FIG. 10, after obtaining the fitness age, the watch may determine whether the fitness age is greater than the chronological age (that is, a process of determining whether the fitness age is greater than the chronological age shown as 1001 in FIG. 10). For two different determination results, the watch may correct the fitness age in a corresponding manner, to improve rationality of the corrected fitness age. Two cases are separately described below.

Case 1: If the fitness age is greater than the chronological age, the watch may lower the fitness age, to make the fitness age appropriately close to the chronological age. Longer exercise duration indicates a smaller corrected fitness age. In this way, for better exercise habits, the fitness age is more likely to be lowered, and therefore, the fitness age may be lowered more significantly.

In a specific implementation, the watch may determine, based on the exercise duration, a correction factor for correcting a target probability (a process of determining a correction factor for a target probability based on the exercise duration, shown as 1002 in FIG. 10), where the target probability is a corresponding probability value of the target VO2max in a probability distribution. For example, the probability distribution is [20(k1), 21(k2), 22(k3), ... 40 (k21) ... 90(k71)], the target VO2max is 40 mL/(kg min), and the target probability p is k21.

If the exercise duration is longer, a smaller correction factor may be determined; or if the exercise duration is shorter, a larger correction factor may be determined. A smaller correction factor indicates a lower corrected target probability obtained by correcting the target probability. A larger correction factor indicates a higher corrected target probability obtained by correcting the target probability.

For example, the watch may determine the correction factor to be a first factor if the exercise duration of the user during a first preset time interval (for example, last 1 week, 14 days, 15 days, ...) is less than the first preset duration. The watch may determine the correction factor to be a second factor if the exercise duration of the user during the first preset time interval is greater than the second preset duration. The first preset duration is equal to the second preset duration. The first factor is greater than the second factor, and both the first factor and the second factor are in a range of [-1, 1]. For the same fitness age, a fitness age corrected by using the first factor is greater than a fitness age corrected by using the second factor.

For example, the first preset time interval is 1 week, and the first preset duration and the second preset duration are each 120 min. If the exercise duration of the user in 1 week is less than 120 min, the correction factor may be determined to be the first factor, for example, 0.5. If the exercise duration of the user in 1 week is greater than 120 min, the correction factor may be determined to be the second factor, for example, -0.5.

In another example, the first preset duration may alternatively be less than the second preset duration. Further, based on the first factor and the second factor, the watch may determine the correction factor to be a third factor if the exercise duration of the user during the first preset time interval is greater than the first preset duration and less than the second preset duration. A correspondence between the exercise duration and the correction factor is shown in Table 4 below.

**Table 4**

| Exercise duration T | Correction factor |
|---|---|
| T<first preset duration | First factor |
| First preset duration<T<second preset duration | Third factor |
| T>second preset duration | Second factor |

The third factor is less than the first factor and greater than the second factor, and the third factor is also in a range of [-1, 1]. For the same fitness age, a fitness age corrected by using the third factor is less than the fitness age corrected by using the first factor and greater than the fitness age corrected by using the second factor.

For example, the first preset time interval is 1 week, the first preset duration is 90 min, and the second preset duration is 150 min. If the exercise duration of the user in 1 week is less than 90 min, the correction factor may be determined to be the first factor, for example, 0.5. If the exercise duration of the user in 1 week is greater than 90 min and less than 150 min, the correction factor may be determined to be the third factor, for example, -0.05. If the exercise duration of the user in 1 week is greater than 150 min, the correction factor may be determined to be the second factor, for example, -0.5.

After obtaining the correction factor, the watch may adjust the target probability based on the correction factor, and increase the chronological age by using the adjusted target probability, to obtain a corrected fitness age (that is, a process of increasing the chronological age based on the correction factor and the target probability, shown as in 1003 in FIG. 10).

For the same target probability, a corrected target probability corrected by using the first factor is greater than a corrected target probability corrected by using the third factor; and the corrected target probability corrected by using the third factor is greater than a corrected target probability corrected by using the second factor.

For example, the corrected target probability p'=p×(l+n1), where p is the target probability before correction, and n1 is the correction factor. For example, n1 is the first factor 0.5, and in this case, the corrected target probability p1' is equal to 1.5p. In another example, n1 is the second factor -0.5, and in this case, the corrected target probability p2' is equal to 0.5p. It is clear that p1' is greater than p2'.

In a specific implementation, after the corrected target probability is obtained, the watch may calculate the corrected fitness age by using Formula (2) below: $age 1 ′ = age 0 + \left(age0-age1\right) \times p ′$ Formula (2)

age1' represents the corrected fitness age, age0 represents the chronological age, age1 represents the fitness age, n1 represents the correction factor, and p' represents the corrected target probability.

The effect of the corrected fitness age is described below by an example in which age1 is equal to 45 years, age0 is equal to 30 years, p is equal to 0.2, the first factor is 0.5, the second factor is - 0.5, and the third factor is - 0.05. Specifically, a correspondence between the exercise duration of the user, the correction factor used, and age1' obtained through correction is shown in Table 5 below:

**Table 5**

| Exercise duration T | Correction factor | age1' (unit: years old) |
|---|---|---|
| T<first preset duration | First factor (0.5) | age1'=30+(45-30)×0.2×(1+0.5)=34.5 |
| First preset duration <T<second preset duration | Third factor (-0.05) | age1'=30+(45-30)×0.2×(1-0.05)=32.85 |
| T>second preset duration | Second factor (-0.5) | age1'=30+(45-30)×0.2×(1-0.5)=31.5 |

As reflected in Table 5, if the exercise duration is longer, a smaller correction factor is used, and the corrected fitness age age1' finally obtained is smaller.

Certainly, in some other implementations, after the correction factor is obtained, the fitness age may be corrected in another manner, which is not limited to the foregoing manner of using Formula (2). For example, the watch may further multiply the fitness age with the correction factor plus one, to obtain a corrected fitness age. That is, the corrected fitness age age1'=age1×(1+n1). In this case, if the exercise duration of the user is longer, a smaller correction factor n1 is used, and the corrected fitness age age1' is smaller. In this way, for better exercise habits, the fitness age may be corrected to obtain a smaller fitness age.

Case 2: If the fitness age is less than the chronological age, the watch may lower or increase the fitness age, so that the longer the exercise duration, the smaller the corrected fitness age. In this way, for better exercise habits, the corrected fitness age becomes smaller.

In a specific implementation, the watch can determine corrected years for correcting the fitness age based on the exercise duration (a process of determining corrected years of the fitness age based on the exercise duration, shown as 1004 in FIG. 10), and then add corresponding corrected years based on the fitness age (a process of increasing or lowering the fitness age based on the corrected years, shown as 1005 in FIG. 10), to obtain the corrected fitness age.

The watch may determine the corrected years to be a first number of years if the exercise duration of the user during a first preset time interval (for example, last 1 week, 14 days, 15 days, ...) is less than the first preset duration. The watch may determine the corrected years to be a second number of years if the exercise duration of the user during the first preset time interval is greater than the first preset duration and less than the second preset duration. The watch may determine the corrected years to be a third number of years if the exercise duration of the user during the first preset time interval is greater than the second preset duration. The first number of years is greater than the second number of years, the second number of years is greater than the third number of years, and the first number of years, the second number of years, and the third number of years are all in a range of [-2, 2]. For the same fitness age, a fitness age corrected by using the first number of years is greater than a fitness age corrected by using the second number of years, and the fitness age corrected by using the second number of years is greater than a fitness age corrected by using the third number of years.

For example, both the first number of years and the second number of years are both positive, and the third number of years is negative. When the watch corrects the fitness age, corrected years may be added based on the fitness age. For example, the first number of years is 2 years, the second number of years is 0.5 years, the third number of years is - 1 years, and the first years is 33 years old. A correspondence between the exercise duration of the user, the corrected years used, and the corrected fitness age age1' is shown in Table 6 below:

**Table 6**

| Exercise duration T | Corrected years | age1' (years old) |
|---|---|---|
| T<first preset duration | First age (2 years old) | age1'=33+2=35 |
| First preset duration <T<second preset duration | Second age (0.5 years old) | age1'=33+0.5=33.5 |
| T>second preset duration | Third age (-1 years old) | age1'=33-1=32 |

As reflected in Table 6, if the exercise duration is longer, the corrected fitness age age1' finally obtained is smaller. In addition, when T<first preset duration, that is, the exercise duration is short, it is considered that the fitness age of the user is more likely to be increased, and large corrected years, such as, 2 years are added, so that the fitness age is increased by a large value. When the first preset duration<T<second preset duration, that is, the exercise duration is normal, it is considered that the fitness age of the user is increased with the chronological age, and small corrected years, such as 0.5 years are added, so that the fitness age is increased by a small value. When T<second preset duration, that is, the exercise duration is long, it is considered that the fitness age of the user is more likely to be decreased, and negative corrected years are added, to decrease the fitness age. In this way, correction on the fitness age is more proper.

During actual implementation, a correction coefficient may also be used to replace the corrected years. For example, if T<first preset duration, the correction factor may be determined to be a first coefficient, where the first coefficient is greater than 1, for example, 1.1. The fitness age is multiplied by the first coefficient, to obtain an increased fitness age. If first preset duration<T<second preset duration, the correction coefficient may be determined to be a second coefficient, where the second coefficient is greater than 1 but less than the first coefficient, for example, 1.05. The fitness age is multiplied by the second coefficient to obtain an increased fitness age, but an increase amplitude is small. If T<second preset duration, the correction factor may be determined to be a third coefficient, where the third coefficient is less than 1, for example, 0.95. The fitness age is multiplied by the third coefficient, to obtain a decreased fitness age. This is not specifically limited in this embodiment of this application.

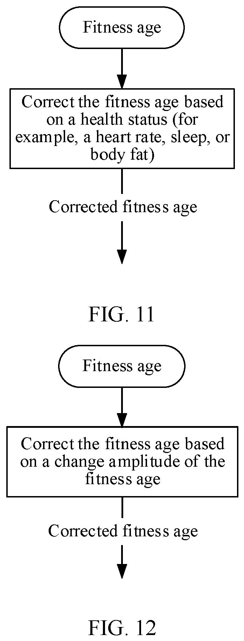

In some other embodiments, referring to FIG. 11, after obtaining the fitness age, the watch may further correct the fitness age based on a health status (for example, a heart rate, sleep, or body fat), to obtain a corrected fitness age. When the health status of the user shows a trend of improvement, the fitness age is lowered; or when the health status shows a trend of worsening, the fitness age is increased. In this way, the corrected fitness age matches the health status of the user.

In a specific implementation, if a resting heart rate of the user during a second preset time interval (for example, 1 week, 14 days, 15 days, ...) shows a trend of decreasing, it indicates that the health status of the user becomes better, and the watch may lower the fitness age by n2 years; or if a resting heart rate of the user during a second preset time interval shows a trend of increasing, it indicates that the health status of the user becomes worse, and the watch may increase the fitness age by n2 years.

In another specific implementation, if a maximum heart rate of the user during a second preset time interval shows a trend of increasing, it indicates that the health status of the user becomes better, and the watch may lower the fitness age by n3 years; or if a maximum heart rate of the user during a second preset time interval shows a trend of decreasing, it indicates that the health status of the user becomes worse, and the watch may increase the fitness age by n3 years.

In another specific implementation, if sleep quality of the user during a second preset time interval shows a trend of improvement, it indicates that the health status of the user becomes better, and the watch may lower the fitness age by n4 years; or if sleep quality of the user during a second preset time interval shows a trend of worsening, it indicates that the health status of the user becomes worse, and the watch may increase the fitness age by n4 years.

In another specific implementation, if a body fat percentage of the user during a second preset time interval shows a trend of decreasing, it indicates that the health status of the user becomes better, and the watch may lower the fitness age by n5 years; or if a body fat percentage of the user during a second preset time interval shows a trend of increasing, it indicates that the health status of the user becomes worse, and the watch may increase the fitness age by n5 years.

n2, n3, n4, and n5 are all in a range of (0, 2]. For example, n2=1. When the resting heart rate becomes lower, the fitness age may be lowered by 1 year; or when the resting heart rate becomes higher, the fitness age may be increased by 1 year.

It is to be noted that the foregoing descriptions respectively describes specific implementations of correcting the fitness age based on various health status parameters. During actual implementation, at least two of the foregoing implementations may be used in combination. This can avoid improper correction on the fitness age due to the fact that a single health status indicator is too one-sided.

The health status parameters such as the resting heart rate, the maximum heart rate, the sleep quality, and the body fat percentage may be routinely detected through a sensor in the watch and stored in the watch. Alternatively, after detecting the health status parameters, the sensor in the watch may send the health status parameters to another electronic device for storage, When the watch needs to use the health status parameters, the health status parameters are obtained from the another electronic device. In this way, storage pressure of the watch may be reduced.

Generally, the fitness age of the user may not greatly change in a short period of time. For example, a case in which the fitness age in three days is 33 years old and the fitness age is 25 years old today may not occur. Based on this, in some embodiments, referring to FIG. 12, after obtaining a fitness age, the watch may further correct, based on a fitness age in the third preset time interval (for example, the last week, half month, or the like), the current fitness age obtained through calculation (that is, correcting the fitness age based on a change amplitude of the fitness age as shown in FIG. 12), to obtain a corrected fitness age. Therefore, the fitness age does not greatly change in a short period of time.

To be specific, the watch may calculate difference between a current fitness age and a plurality of fitness ages in a third preset time interval (for example, the last week, half month, or the like), and determine whether a maximum difference exceeds a preset difference, for example, 3 years, 5 years, or the like. If the maximum difference does not exceed the preset difference, no processing may be performed. If the maximum difference exceeds the preset difference, it indicates that a change amplitude in the fitness age in a short period of time is excessive, and the fitness age is corrected, so that the differences between the current fitness age and the plurality of fitness ages in the third preset time interval (for example, the last week, half month, or the like) is in a range of the preset difference.

For example, the preset difference is 3 years, the current fitness age is 33 years old, and the plurality of fitness ages (which may be corrected fitness ages) obtained in the last week include 30 years old, 31 years old, 30 years old, 29 years old, and 32 years old. The maximum difference between the current fitness age and the plurality of fitness ages in the last week is 33-29=4 years, and obviously greater than 3 years, and in this case, the current fitness age may be corrected to 32 years old, so that the maximum difference does not exceed 3 years.

It is to be noted that the descriptions separately provide correction solutions based on the exercise duration, the health status, and the change amplitude in the fitness age with reference to FIG. 9 to FIG. 12. During actual implementation, at least two of the foregoing solutions may be used in combination. For example, after obtaining the fitness age, the watch may first correct the fitness age by using the embodiment shown in FIG. 9. After correction performed by using the embodiment in FIG. 9 is completed, the fitness age may be corrected by using the embodiments in FIG. 11 and FIG. 12 sequentially, to finally obtain the corrected fitness age.

It may be understood that in the embodiment in which the fitness age is corrected, the fitness age displayed by the watch is the corrected fitness age.

In some scenarios, the fitness age obtained through mapping may be very improper, for example, the fitness age exceeds a normal fitness age range by 18 to 65 years old. Based on this, if the fitness age is further corrected by using the embodiments shown in FIG. 9 to FIG. 12, there may be significant deviations in both a determined correction scheme and a correction result due to a severely improper correction basis.

Based on this, in some embodiments, to improve rationality of correction on the fitness age, before the fitness age is corrected by using the embodiments shown in FIG. 9 to FIG. 12, the watch may first detect whether the fitness age is in a proper fitness age range (or may be referred to as a preset age range). If the fitness age is in the proper fitness age range, the watch may further correct the fitness age. If the fitness age is not in the proper fitness age range, the watch may directly adjust the fitness age to be in the proper age range. For example, the proper fitness age range is 20-65 years old, and if the fitness age obtained through mapping is 75 years old, that is, 65 years old or above, the watch may adjust the fitness age to 65 years old. Further, after the fitness age is adjusted to be in the proper age range, the watch may further correct the fitness age by using the embodiments in FIG. 9 to FIG. 12.

For example, before the fitness age is corrected by using Formula (2), the watch may detect whether the fitness age is in the proper fitness age range, and when the fitness age is in the proper fitness age range, the fitness age is further corrected by using Formula (2). If the fitness age is not in the proper fitness age range, the fitness age may be directly adjusted to be in the proper age range, and a process of correcting the fitness age by using Formula (2) is omitted. However, subsequent correction on the fitness age is performed still in the manner shown in FIG. 11 and FIG. 12.

To help the user be more accurately aware of a level of the fitness age (either the fitness age obtained through mapping or the corrected fitness age), after the fitness age is obtained, the watch may further determine a level of the fitness age of the user in peers and provide the level for the user. For example, a prompt of "Go beyond 50% of peers" shown in FIG. 2 is displayed to indicate that the fitness age of the user is superior to 50% of peers.

The watch may determine a target age bracket of the user based on the chronological age of the user. Matching is performed on VO2max of the user and ranges of VO2max (or may be referred to as first oxygen uptake ranges) corresponding to a plurality of levels in the target age bracket, and the level of the fitness age of the user is determined.

For example, the age bracket includes 6 ranges of 18-25 years old, 26-35 years old, 36-45 years old, 46-55 years old, 56-65 years old, and 65 years old or above shown in Table 1, and each age bracket includes 7 levels, namely, "excellent", "good", "above average", "average", "below average", "poor", and "very poor". The age bracket of 26-35 years old is used as an example, and ranges of VO2max corresponding to the 7 levels are shown in Table 7 below:

**Table 7**

| | 26-35 years old | |
|---|---|---|
| Level | Male | Female |
| Excellent | 60-95 mL/(kg min) | 58-95 mL/(kg min) |
| Good | 50-55 mL/(kg min) | 48-53 mL/(kg min) |
| Above average | 44-48 mL/(kg min) | 43-45 mL/(kg min) |
| Average | 39-42 mL/(kg min) | 37-41 mL/(kg min) |
| Below average | 34-38 mL/(kg min) | 34-36 mL/(kg min) |
| Poor | 30-33 mL/(kg min) | 28-32 mL/(kg min) |
| Very poor | 15-27 mL/(kg min) | 14-25 mL/(kg min) |

As reflected in Table 7, for males aged 26-35 years old, a range of VO2max corresponding to the "excellent" level of the fitness age is 60-95 mL/(kg min), a range of VO2max corresponding to the "good" level of the fitness age is 50-55 mL/(kg min), ..., and a range of VO2max corresponding to the "very poor" level of the fitness age is 15-27 mL/(kg min). For females aged 26-35 years old, a range of VO2max corresponding to the "excellent" level of the fitness age is 58-95 mL/(kg min), a range of VO2max corresponding to the "good" level of the fitness age is 48-53 mL/(kg min), ..., and a range of VO2max corresponding to the "very poor" level of the fitness age is 14-25 mL/(kg min).

If the user is a female having a chronological age between 26-35 years, such as a female of 30 years old, and VO2max calculated based on the test exercise is 50 mL/(kg min), where 50 mL/(kg min) falls within the range of 48-53 mL/(kg min) corresponding to the "good" level, it is determined that the level of the fitness age of the female of 30 years old is "good". When displaying the fitness age, the watch may simultaneously prompt the user for the level of fitness age, such as "good", "very good", "go beyond 80% of peers", and the like.

Generally, when the fitness age is less than the chronological age, it indicates that the fitness age of the user is at a relatively excellent level, and in this case, the user is prompted about the level of the fitness age, to give the user positive feedback. Based on this, in some embodiments, the watch may further determine the level of the fitness age and provide the fitness age for the user when determining that the fitness age is less than the chronological age, that is, after determining that the fitness age is less than the chronological age, shown as 1001 in FIG. 10.

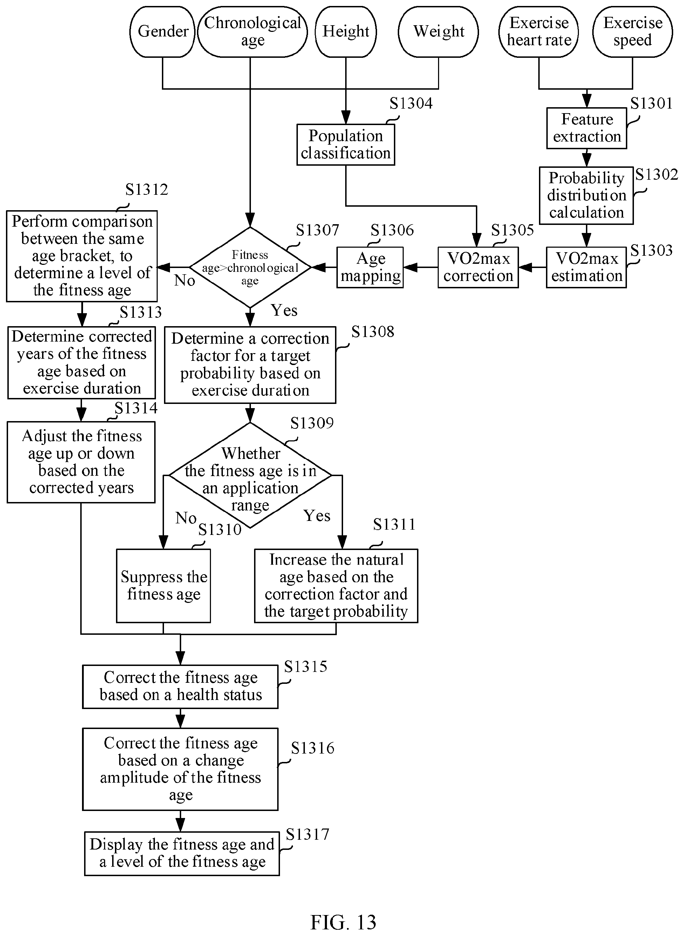

To facilitate understanding of embodiments of this application, descriptions are made below with reference to the complete example shown in FIG. 13.

First, the maximal oxygen uptake, that is, VO2max, is calculated.

S1301. Perform feature extraction.

A heart rate feature is extracted from the exercise heart rate and a speed feature is extracted from the exercise speed for calculating VO2max. For details, reference may be made to related descriptions of 601 shown in FIG. 6.

S1302. Perform probability distribution calculation.

Probabilities of all possible values of VO2max are calculated. For details, reference may be made to related descriptions of 602 shown in FIG. 6.

S1303. Perform VO2max estimation.

For example, VO2max is obtained by performing weighted calculation. For details, reference may be made to related descriptions of 602 shown in FIG. 6.

After VO2max is obtained, VO2max may be corrected based on population classifications. S1304 and S1305 are described below.

S1304. Perform population classification.

The target population to which the user belongs may be determined based on the gender, the chronological age, the height, the weight, and the like. For details, reference may be made to related descriptions of FIG. 7.

S1305. Perform VO2max correction.

If VO2max calculated is not in a proper range of VO2max of the target population, VO2max may be corrected to the proper range of VO2max of the target population, so that VO2max is in a proper range. For details, reference may be made to descriptions of 701 and 702 shown in FIG. 7.

S1306. Perform Age mapping.

A mapping relationship between a fitness age range corresponding to the gender of the user and the range of VO2max is queried, to determine a fitness age matching the target VO2max (VO2max obtained in S1303 or S1306). For example, the mapping relationship shown in Table 3 may be queried, and an age having a mapping relationship with an oxygen uptake having the smallest difference from VO2max may be used as the fitness age. For details, reference may be made to descriptions of 801 shown in FIG. 8.

After the fitness age is determined, the fitness age may be further corrected to make the fitness age more proper. Manners for correction include: correcting the fitness age based on the exercise duration, correcting the fitness age based on the health status, correcting the fitness age based on the change amplitude of the fitness age, and correcting the fitness age based on the range of the fitness age, and the like.

S1307. Determine whether the fitness age is greater than the chronological age. If the fitness age is greater than the chronological age, S1308 is performed; or if the fitness age is not greater than the chronological age, S1312 is performed.

If the fitness age is greater than the chronological age, the chronological age is increased by using a target probability, to correct the fitness age. S 1308 to S1311 are described below.

S1308. Determine a correction factor for a target probability based on exercise duration.

If the exercise duration is longer, a smaller correction factor is determined for increasing the chronological age in a smaller magnitude, to obtain a smaller corrected fitness age. If the exercise duration is shorter, a larger correction factor is determined for increasing the chronological age in a larger magnitude, to obtain a larger corrected fitness age. For details, reference may be made to descriptions of 1002 shown in FIG. 10.

In some embodiments, for the case that the fitness age is greater than the chronological age, to avoid an improper situation due to an excessive fitness age, rationality of the fitness age may be detected, and a corresponding correction strategy is adopted based on a detection result. S1309 to S1311 are described below.

S1309. Determine whether the fitness age is in an applicable range. If yes, S1311 is executed, and if no, S1310 is executed.

For example, whether the fitness age exceeds 65 years old is detected, and if the fitness age exceeds 65 years old, it is not considered that the fitness age is in an applicable range is not in the applicable range.

S1310. Suppress the fitness age.

The fitness age is adjusted to be in a proper age range, for example, in a range of 18-65 years old.

For specific implementations of S1309 and S1310, reference may be made to the descriptions of the relevant part above.

S1311. Increase the chronological age based on the correction factor and the target probability.

For example, the chronological age is increased by using Formula (2), to obtain a corrected fitness age. For details, reference may be made to related descriptions of 1003 shown in FIG. 10.

If the fitness age is less than the chronological age, the level of the fitness age may be determined obtain a positive feedback result, as shown in S1312 below, or the fitness age may be corrected based on the exercise duration, as shown in S1313 and S1314 below.

S1312. Perform comparison between the same age bracket, to determine a level of the fitness age.

For example, the level of the fitness age is determined by using ranges of VO2max corresponding to a plurality of levels in the age bracket similar to that shown in Table 7. For details, reference may be made to the descriptions above and below Table 7.

S1313: Determine a correction factor for a fitness age based on exercise duration.

S1314: Increase or lower the fitness age based on the corrected years.

For specific implementation of S1313 and S1314, reference may be made to the related descriptions of 1004 and 1005 shown in FIG. 10.

Further, correction on the fitness age further includes: correcting the fitness age to match the health status of the user, as shown in S1315 below; and correcting the fitness age, so that a change amplitude in the fitness age in a short period of time is not excessively large, as shown in S1316 below.

S1315. Correct the fitness age based on a health status.

If the health status of the user becomes better, the fitness age is lowered. If the health status of the user becomes worse, the fitness age is increased. The health status may be measured by using one or more of the following parameters: a change in the resting heart rate, a change in the maximum heart rate, a change in sleep quality, and a change in the body fat percentage. For details, reference may be made to related descriptions of FIG. 11.

S1316. Correct the fitness age based on a change amplitude of the fitness age.

For example, the fitness age is corrected, so that the change amplitude in the fitness age in the last week does not exceed 3 years. For details, reference may be made to related descriptions of FIG. 12.

S1317. Display the fitness age and a level of the fitness age.

It may be understood that, in this embodiment, the level of the fitness age is determined only for the case that the fitness age is less than the chronological age, and therefore, displayed information is generally positive. For example, the information is "Go beyond 50% of peers", "Your fitness age is excellent", or the like.

Based on the above, in this embodiment of this application, the fitness age may be obtained through mapping based on VO2max and then is displayed. In this way, the fitness age may be estimated after daily exercise is completed. This can routinely obtain a fitness age, and reduce the difficulty in obtaining the fitness age. In addition, the fitness age may be corrected in a plurality of dimensions, to make the fitness age finally obtained more proper.

An embodiment of this application further provides an electronic device. The electronic device may include a memory and one or more processors. The memory is coupled to the processor. The memory is configured to store computer program code, where the computer program code includes computer instructions. When the processor executes the computer instructions, the electronic device can perform the functions or the steps performed by the watch in the foregoing method embodiments, to estimate a fitness age.

An embodiment of this application further provides a communication system, the communication system may include the electronic device (or referred to as a first electronic device) for estimating a fitness age and a second electronic device. The second electronic device is in communication connection to the first electronic device. The second electronic device includes at least a memory. The memory is configured to store data stored in other electronic devices in the foregoing method embodiments. For example, the memory stores data for group classification, a historical probability distribution, a health status parameter, and the like.

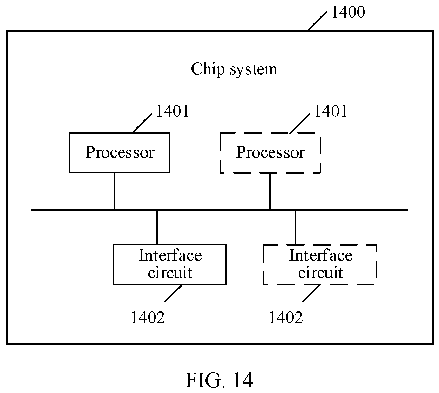

An embodiment of this application further provides a chip system. As shown in FIG. 14, the chip system 1400 includes at least one processor 1401 and at least one interface circuit 1402. The processor 1401 and the interface circuit 1402 may be interconnected through a line. For example, the interface circuit 1402 may be configured to receive a signal from another apparatus (for example, the memory of the electronic device). In another example, the interface circuit 1402 may be configured to send a signal to another apparatus (for example, the processor 1401). For example, the interface circuit 1402 may read instructions stored in the memory and send the instructions to the processor 1401. When the instructions are executed by the processor 1401, the electronic device is enabled to perform the steps in the foregoing embodiments. Certainly, the chip system may further include other discrete devices. This is not specifically limited in this embodiment of this application.

An embodiment of this application further provides a computer-readable storage medium. The computer-readable storage medium stores computer instructions. When the computer instructions are run on an electronic device, the electronic device is enabled to perform the functions or steps performed by the watch in the method embodiments, to estimate a fitness age.

An embodiment of this application further provides a computer program product. When the computer program product is run on a computer, the computer is enabled to perform the functions or steps performed by the watch in the foregoing method embodiments, to estimate a fitness age.

In addition, an embodiment of this application further provides an apparatus. The apparatus may specifically be a chip, a component, or a module. The apparatus may include a processor and a memory that are connected to each other, where the memory is configured to store computer-executable instructions, and when the apparatus operates, the processor may execute the computer-executable instructions stored in the memory, so that the chip performs the functions or steps performed by the watch in the foregoing method embodiments, to estimate a fitness age.

The electronic device, the communication system, the computer-readable storage medium, the computer program product, or the chip provided in embodiments is configured to perform the corresponding method provided above. Therefore, for beneficial effects that can be achieved, refer to the beneficial effects in the corresponding method provided above. Details are not described herein.

Through the descriptions of the foregoing implementations, a person skilled in the art may clearly understand that, for the purpose of convenient and brief description, only division of the foregoing functional modules is used as an example for description. In actual application, the foregoing functions may be allocated to and completed by different functional modules according to requirements. That is, an internal structure of an apparatus is divided into different functional modules to complete all or some of the functions described above.

In the several embodiments provided in this application, it is to be understood that the disclosed apparatus and method may be implemented in another manner. For example, the described apparatus embodiment is merely exemplary. For example, the module or the unit division is merely a logical function division and may be other division during actual implementation. For example, a plurality of units or components may be combined or integrated into another apparatus, or some features may be ignored or not performed. In addition, the displayed or discussed mutual couplings or direct couplings or communication connections may be implemented through some interfaces. The indirect couplings or communication connections between the apparatuses or units may be implemented in electronic, mechanical, or other forms.

The units described as separate parts may or may not be physically separate, and parts displayed as units may be one or more physical units, that is, may be located in one place, or may be distributed in a plurality of different places. Some or all of the units may be selected based on actual requirements to achieve the objectives of the solutions of embodiments.

In addition, functional units in embodiments of this application may be integrated into one processing unit, each of the units may exist alone physically, or two or more units are integrated into one unit. The integrated unit may be implemented in a form of hardware, or may be implemented in a form of a software functional unit.

When the integrated unit is implemented in the form of a software function unit and sold or used as an independent product, the integrated unit may be stored in a readable storage medium. Based on such an understanding, the technical solutions in embodiments of this application essentially, or the part contributing to the related art, or all or some of the technical solutions may be implemented in the form of a software product. The software product is stored in a storage medium and includes several instructions for instructing a device (which may be a single-chip microcomputer, a chip, or the like) or a processor (processor) to perform all or some of the steps of the methods described in embodiments of this application. The foregoing storage medium includes any medium that can store program code, such as a USB flash drive, a removable hard disk, a read-only memory (Read-Only Memory, ROM), a random access memory (Random Access Memory, RAM), a magnetic disk, or an optical disc.

## Claims

1. A fitness age estimation method, comprising:
displaying, by an electronic device, a first interface (101), wherein the first interface (101) comprises a first control (102), and the first control (102) is configured to trigger the electronic device to test a maximal oxygen uptake of a user;
displaying, by the electronic device, test guide information after the electronic device detects a first operation performed by the user on the first control (102), wherein the test guide information is used for guiding the user to complete a test exercise for testing the maximal oxygen uptake; and
displaying, by the electronic device, a second interface (201) after detecting that the test exercise ends, wherein the second interface (201) comprises a fitness age of the user, and the fitness age is obtained by the electronic device by performing mapping based on the maximal oxygen uptake obtained through testing;
wherein the estimation method is **characterized in that**:
after obtaining the fitness age, if the fitness age is greater than a chronological age of the user, correcting (S1311; 1002, 1003), by the electronic device, the fitness age based on exercise duration of the user in a first preset time interval, to obtain a corrected fitness age, wherein longer exercise duration indicates a smaller corrected fitness age; and
that the second interface (201) comprises a fitness age of the user is specifically: the second interface (201) comprises the corrected fitness age.

2. The method according to claim 1, wherein the second interface (201) further comprises indication information, wherein the indication information is used for indicating a level of the fitness age.

3. The method according to claim 2, wherein after the electronic device obtains the maximal oxygen uptake through testing, the method further comprises:
determining, by the electronic device, a level of the maximal oxygen uptake in a first oxygen uptake range corresponding to a target age bracket, to obtain the level of the fitness age, wherein
the target age bracket is an age bracket to which the chronological age of the user belongs.

4. The method according to any one of claims 1 to 3, wherein after it is detected that the test exercise ends, the method further comprises:
displaying, by the electronic device, a third interface, wherein the third interface comprises the fitness age and a second control, and the second control is configured to trigger the electronic device to re-estimate the fitness age;
displaying, by the electronic device, guide information after the electronic device detects a second operation performed by the user on the second control, wherein the guide information is used for guiding the user to complete a test exercise for testing the maximal oxygen uptake; and
displaying, by the electronic device, a fourth interface after detecting that the test exercise ends, wherein the fourth interface comprises an updated fitness age.

5. The method according to any one of claims 1 to 4, wherein the electronic device comprises a mapping relationship between an age and an oxygen uptake; and
that the electronic device obtains the fitness age by performing mapping based on the maximal oxygen uptake obtained through testing comprises:
querying, by the electronic device, the maximal oxygen uptake in the mapping relationship, and determining an age having a mapping relationship with the maximal oxygen uptake as the fitness age.

6. The method according to any one of claims 1 to 5, wherein that the electronic device tests the maximal oxygen uptake of the user comprises:
calculating, by the electronic device, a probability distribution of the maximal oxygen uptake based on an exercise heart rate and an exercise speed of the user during the test exercise; and
performing, by the electronic device, weighted summation on an oxygen uptake and a probability corresponding to the oxygen uptake that are comprised in the probability distribution, to obtain the maximal oxygen uptake.

7. The method according to claim 6, wherein the correcting (S1311; 1002, 1003), by the electronic device, the fitness age based on exercise duration of the user in the first preset time interval, to obtain the corrected fitness age comprises:
if the fitness age is greater than the chronological age of the user, correcting, by the electronic device, the fitness age by using the following formula: $age 1 ′ = age 0 + \left(age0-age1\right) \times p \times \left(1+n1\right)$
wherein age1' represents the corrected fitness age, age0 represents the chronological age, age1 represents the fitness age, p represents a target probability corresponding to the maximal oxygen uptake in the probability distribution, and n1 represents a correction factor, wherein longer exercise duration indicates a smaller correction factor, and n1 is in a range of [-1, 1].

8. The method according to any one of claims 1 to 7, wherein after the electronic device obtains the fitness age through mapping, the method further comprises:
correcting (S1315), by the electronic device, the fitness age based on a change in a health status of the user in second preset time, to obtain the corrected fitness age, wherein
when the health status shows a trend of improvement, the electronic device lowers the fitness age; or when the health status shows a trend of worsening, the electronic device increases the fitness age.

9. The method according to claim 8, wherein the correcting (S1315), by the electronic device, the fitness age based on the change in the health status of the user in second preset time, to obtain the corrected fitness age comprises at least one of the following:
if a resting heart rate of the user in a second preset time interval shows a trend of decreasing, the electronic device lowers the fitness age by n2 years; or if a resting heart rate of the user in a second preset time interval shows a trend of increasing, the electronic device increases the fitness age by n2 years;
if a maximum heart rate of the user in a second preset time interval shows a trend of increasing, lowering, by the electronic device, the fitness age by n3 years; or if a maximum heart rate the user in a second preset time interval shows a trend of decreasing, increasing, by the electronic device, the fitness age by n3 years;
if sleep quality of the user in a second preset time interval shows a trend of improvement, lowering, by the electronic device, the fitness age by n4 years; or if sleep quality of the user in a second preset time interval shows a trend of worsening, increasing, by the electronic device, the fitness age by n4 years; or
if a body fat percentage of the user in a second preset time interval shows a trend of decreasing, the electronic device lowers the fitness age by n5 years; or if a body fat percentage of the user in a second preset time interval shows a trend of increasing, the electronic device increases the fitness age by n5 years.

10. The method according to any one of claims 1 to 9, wherein after the electronic device obtains the fitness age through mapping, the method further comprises:
if a difference between the fitness age and any historical fitness age in a third preset time interval exceeds a preset difference, correcting, by the electronic device, the fitness age, to obtain a corrected fitness age, wherein a difference between the corrected fitness age and any historical fitness age in the third preset time interval does not exceed the preset difference.

11. The method according to any one of claims 6 to 10, wherein the correcting the fitness age comprises:
if the fitness age is in a preset age range, correcting the fitness age; and
if the fitness age is not in the preset age range, adjusting, by the electronic device, the fitness age to be in the preset age range, and correcting the fitness age.

12. An electronic device, wherein the electronic device comprises a memory (521) and a processor (510), the memory (521) is coupled to the processor (510), the memory (521) stores computer program code, the computer program code comprises computer instructions, and when the computer instructions are executed by the processor (510), the electronic device is enabled to perform the method according to any one of claims 1 to 11.

13. A computer-readable storage medium, comprising computer instructions, wherein when the computer instructions are run on an electronic device, the electronic device is enabled to perform the method according to any one of claims 1 to 11.

## Patentansprüche

1. Fitnessalter-Schätzverfahren, umfassend:
Anzeigen, durch eine elektronische Vorrichtung, einer ersten Schnittstelle (101), wobei die erste Schnittstelle (101) ein erstes Steuerelement (102) umfasst und das erste Steuerelement (102) dazu eingerichtet ist, die elektronische Vorrichtung dazu zu veranlassen, eine maximale Sauerstoffaufnahme eines Benutzers zu testen;
Anzeigen, durch die elektronische Vorrichtung, von Testführungsinformationen, nachdem die elektronische Vorrichtung eine durch den Benutzer an dem ersten Steuerelement (102) durchgeführte erste Operation erfasst hat, wobei die Testführungsinformationen dazu verwendet werden, den Benutzer anzuleiten, eine Testübung zum Testen der maximalen Sauerstoffaufnahme abzuschließen; und
Anzeigen, durch die elektronische Vorrichtung, einer zweiten Schnittstelle (201) nach Erfassen, dass die Testübung beendet ist, wobei die zweite Schnittstelle (201) ein Fitnessalter des Benutzers umfasst und das Fitnessalter durch die elektronische Vorrichtung durch Durchführen einer Abbildung basierend auf der durch Testen erhaltenen maximalen Sauerstoffaufnahme erhalten wird;
wobei das Schätzverfahren **dadurch gekennzeichnet ist, dass**:
nach Erhalten des Fitnessalters, wenn das Fitnessalter größer als ein chronologisches Alter des Benutzers ist, Korrigieren (S1311; 1002, 1003), durch die elektronische Vorrichtung, des Fitnessalters basierend auf einer Trainingsdauer des Benutzers in einem ersten voreingestellten Zeitintervall, um ein korrigiertes Fitnessalter zu erhalten, wobei eine längere Trainingsdauer ein kleineres korrigiertes Fitnessalter angibt; und
dass die zweite Schnittstelle (201) ein Fitnessalter des Benutzers umfasst, spezifisch bedeutet, dass die zweite Schnittstelle (201) das korrigierte Fitnessalter umfasst.

2. Verfahren nach Anspruch 1, wobei die zweite Schnittstelle (201) ferner Angabeinformationen umfasst, wobei die Angabeinformationen verwendet werden, um ein Niveau des Fitnessalters anzugeben.

3. Verfahren nach Anspruch 2, wobei, nachdem die elektronische Vorrichtung die maximale Sauerstoffaufnahme durch Testen erhalten hat, das Verfahren ferner umfasst:
Bestimmen, durch die elektronische Vorrichtung, eines Niveaus der maximalen Sauerstoffaufnahme in einem ersten Sauerstoffaufnahmebereich entsprechend einer Zielaltersgruppe, um das Niveau des Fitnessalters zu erhalten, wobei
die Zielaltersgruppe eine Altersgruppe ist, zu der das chronologische Alter des Benutzers gehört.

4. Verfahren nach einem der Ansprüche 1 bis 3, wobei, nachdem erfasst wird, dass die Testübung beendet ist, das Verfahren ferner umfasst:
Anzeigen, durch die elektronische Vorrichtung, einer dritten Schnittstelle, wobei die dritte Schnittstelle das Fitnessalter und ein zweites Steuerelement umfasst und das zweite Steuerelement dazu eingerichtet ist, die elektronische Vorrichtung dazu zu veranlassen, das Fitnessalter neu zu schätzen;
Anzeigen, durch die elektronische Vorrichtung, von Führungsinformationen, nachdem die elektronische Vorrichtung eine durch den Benutzer an dem zweiten Steuerelement durchgeführte zweite Operation erfasst hat, wobei die Führungsinformationen dazu verwendet werden, den Benutzer anzuleiten, eine Testübung zum Testen der maximalen Sauerstoffaufnahme abzuschließen; und
Anzeigen, durch die elektronische Vorrichtung, einer vierten Schnittstelle nach Erfassen, dass die Testübung beendet ist, wobei die vierte Schnittstelle ein aktualisiertes Fitnessalter umfasst.

5. Verfahren nach einem der Ansprüche 1 bis 4, wobei die elektronische Vorrichtung eine Abbildungsbeziehung zwischen einem Alter und einer Sauerstoffaufnahme umfasst; und
dass die elektronische Vorrichtung das Fitnessalter durch Durchführen einer Abbildung basierend auf der durch Testen erhaltenen maximalen Sauerstoffaufnahme erhält, umfasst:
Abfragen, durch die elektronische Vorrichtung, der maximalen Sauerstoffaufnahme in der Abbildungsbeziehung, und Bestimmen eines Alters, das eine Abbildungsbeziehung mit der maximalen Sauerstoffaufnahme aufweist, als das Fitnessalter.

6. Verfahren nach einem der Ansprüche 1 bis 5, wobei, dass die elektronische Vorrichtung die maximale Sauerstoffaufnahme des Benutzers testet, umfasst:
Berechnen, durch die elektronische Vorrichtung, einer Wahrscheinlichkeitsverteilung der maximalen Sauerstoffaufnahme basierend auf einer Trainingsherzfrequenz und einer Trainingsgeschwindigkeit des Benutzers während der Testübung; und
Durchführen, durch die elektronische Vorrichtung, einer gewichteten Summierung an einer Sauerstoffaufnahme und einer der Sauerstoffaufnahme entsprechenden Wahrscheinlichkeit, die in der Wahrscheinlichkeitsverteilung enthalten sind, um die maximale Sauerstoffaufnahme zu erhalten.

7. Verfahren nach Anspruch 6, wobei das Korrigieren (S1311; 1002, 1003), durch die elektronische Vorrichtung, des Fitnessalters basierend auf einer Trainingsdauer des Benutzers in dem ersten voreingestellten Zeitintervall, um das korrigierte Fitnessalter zu erhalten, umfasst:
wenn das Fitnessalter größer als das chronologische Alter des Benutzers ist, Korrigieren, durch die elektronische Vorrichtung, des Fitnessalters unter Verwendung der folgenden Formel: $age 1 ' = age 0 + \left(age0-age1\right) \times p \times \left(1+n1\right)$
wobei age1' das korrigierte Fitnessalter darstellt, age0 das chronologische Alter darstellt, age1 das Fitnessalter darstellt, p eine Zielwahrscheinlichkeit darstellt, die der maximalen Sauerstoffaufnahme in der Wahrscheinlichkeitsverteilung entspricht, und n1 einen Korrekturfaktor darstellt, wobei eine längere Trainingsdauer einen kleineren Korrekturfaktor angibt und n1 in einem Bereich von [-1, 1] liegt.

8. Verfahren nach einem der Ansprüche 1 bis 7, wobei, nachdem die elektronische Vorrichtung das Fitnessalter durch Abbildung erhalten hat, das Verfahren ferner umfasst:
Korrigieren (S1315), durch die elektronische Vorrichtung, des Fitnessalters basierend auf einer Änderung eines Gesundheitszustands des Benutzers in einer zweiten voreingestellten Zeit, um das korrigierte Fitnessalter zu erhalten, wobei
wenn der Gesundheitszustand einen Trend zur Verbesserung zeigt, die elektronische Vorrichtung das Fitnessalter senkt; oder wenn der Gesundheitszustand einen Trend zur Verschlechterung zeigt, die elektronische Vorrichtung das Fitnessalter erhöht.

9. Verfahren nach Anspruch 8, wobei das Korrigieren (S1315), durch die elektronische Vorrichtung, des Fitnessalters basierend auf der Änderung des Gesundheitszustands des Benutzers in der zweiten voreingestellten Zeit, um das korrigierte Fitnessalter zu erhalten, mindestens eines von Folgendem umfasst:
wenn eine Ruheherzfrequenz des Benutzers in einem zweiten voreingestellten Zeitintervall einen Trend zur Verringerung zeigt, die elektronische Vorrichtung das Fitnessalter um n2 Jahre senkt; oder wenn eine Ruheherzfrequenz des Benutzers in einem zweiten voreingestellten Zeitintervall einen Trend zur Erhöhung zeigt, die elektronische Vorrichtung das Fitnessalter um n2 Jahre erhöht;
wenn eine maximale Herzfrequenz des Benutzers in einem zweiten voreingestellten Zeitintervall einen Trend zur Erhöhung zeigt, Senken, durch die elektronische Vorrichtung, des Fitnessalters um n3 Jahre; oder wenn eine maximale Herzfrequenz des Benutzers in einem zweiten voreingestellten Zeitintervall einen Trend zur Verringerung zeigt, Erhöhen, durch die elektronische Vorrichtung, des Fitnessalters um n3 Jahre;
wenn eine Schlafqualität des Benutzers in einem zweiten voreingestellten Zeitintervall einen Trend zur Verbesserung zeigt, Senken, durch die elektronische Vorrichtung, des Fitnessalters um n4 Jahre; oder wenn eine Schlafqualität des Benutzers in einem zweiten voreingestellten Zeitintervall einen Trend zur Verschlechterung zeigt, Erhöhen, durch die elektronische Vorrichtung, des Fitnessalters um n4 Jahre; oder
wenn ein Körperfettanteil des Benutzers in einem zweiten voreingestellten Zeitintervall einen Trend zur Verringerung zeigt, die elektronische Vorrichtung das Fitnessalter um n5 Jahre senkt; oder wenn ein Körperfettanteil des Benutzers in einem zweiten voreingestellten Zeitintervall einen Trend zur Erhöhung zeigt, die elektronische Vorrichtung das Fitnessalter um n5 Jahre erhöht.

10. Verfahren nach einem der Ansprüche 1 bis 9, wobei, nachdem die elektronische Vorrichtung das Fitnessalter durch Abbildung erhalten hat, das Verfahren ferner umfasst:
wenn eine Differenz zwischen dem Fitnessalter und einem beliebigen historischen Fitnessalter in einem dritten voreingestellten Zeitintervall eine voreingestellte Differenz überschreitet, Korrigieren, durch die elektronische Vorrichtung, des Fitnessalters, um ein korrigiertes Fitnessalter zu erhalten, wobei eine Differenz zwischen dem korrigierten Fitnessalter und einem beliebigen historischen Fitnessalter in dem dritten voreingestellten Zeitintervall die voreingestellte Differenz nicht überschreitet.

11. Verfahren nach einem der Ansprüche 6 bis 10, wobei das Korrigieren des Fitnessalters umfasst:
wenn das Fitnessalter in einem voreingestellten Altersbereich liegt, Korrigieren des Fitnessalters; und
wenn das Fitnessalter nicht in dem voreingestellten Altersbereich liegt, Anpassen, durch die elektronische Vorrichtung, des Fitnessalters, sodass es in dem voreingestellten Altersbereich liegt, und Korrigieren des Fitnessalters.

12. Elektronische Vorrichtung, wobei die elektronische Vorrichtung einen Speicher (521) und einen Prozessor (510) umfasst, der Speicher (521) mit dem Prozessor (510) gekoppelt ist, der Speicher (521) Computerprogrammcode speichert, der Computerprogrammcode Computeranweisungen umfasst und, wenn die Computeranweisungen durch den Prozessor (510) ausgeführt werden, die elektronische Vorrichtung dazu befähigt wird, das Verfahren nach einem der Ansprüche 1 bis 11 durchzuführen.

13. Computerlesbares Speichermedium, umfassend Computeranweisungen, wobei, wenn die Computeranweisungen auf einer elektronischen Vorrichtung ausgeführt werden, die elektronische Vorrichtung dazu befähigt wird, das Verfahren nach einem der Ansprüche 1 bis 11 durchzuführen.

## Revendications

1. Procédé d'estimation d'âge de forme physique, comprenant :
l'affichage, par un dispositif électronique, d'une première interface (101), dans lequel la première interface (101) comprend un premier élément de commande (102), et le premier élément de commande (102) est configuré pour déclencher le dispositif électronique afin de tester une consommation maximale d'oxygène d'un utilisateur ;
l'affichage, par le dispositif électronique, d'informations de guidage de test après que le dispositif électronique a détecté une première opération effectuée par l'utilisateur sur le premier élément de commande (102), dans lequel les informations de guidage de test sont utilisées pour guider l'utilisateur pour achever un exercice de test pour tester la consommation maximale d'oxygène ; et
l'affichage, par le dispositif électronique, d'une deuxième interface (201) après avoir détecté que l'exercice de test se termine, dans lequel la deuxième interface (201) comprend un âge de forme physique de l'utilisateur, et l'âge de forme physique est obtenu par le dispositif électronique en effectuant une mise en correspondance sur la base de la consommation maximale d'oxygène obtenue par test ;
dans lequel le procédé d'estimation est **caractérisé en ce que** :
après l'obtention de l'âge de forme physique, si l'âge de forme physique est supérieur à un âge chronologique de l'utilisateur, la correction (S1311 ; 1002, 1003), par le dispositif électronique, de l'âge de forme physique sur la base d'une durée d'exercice de l'utilisateur dans un premier intervalle de temps prédéfini, pour obtenir un âge de forme physique corrigé, dans lequel une durée d'exercice plus longue indique un âge de forme physique corrigé plus petit ; et
le fait que la deuxième interface (201) comprend un âge de forme physique de l'utilisateur consiste spécifiquement **en ce que** : la deuxième interface (201) comprend l'âge de forme physique corrigé.

2. Procédé selon la revendication 1, dans lequel la deuxième interface (201) comprend en outre des informations d'indication, dans lequel les informations d'indication sont utilisées pour indiquer un niveau de l'âge de forme physique.

3. Procédé selon la revendication 2, dans lequel après que le dispositif électronique a obtenu la consommation maximale d'oxygène par test, le procédé comprend en outre :
la détermination, par le dispositif électronique, d'un niveau de la consommation maximale d'oxygène dans une première plage de consommation d'oxygène correspondant à une tranche d'âge cible, pour obtenir le niveau de l'âge de forme physique, dans lequel
la tranche d'âge cible est une tranche d'âge à laquelle appartient l'âge chronologique de l'utilisateur.

4. Procédé selon l'une quelconque des revendications 1 à 3, dans lequel après qu'il a été détecté que l'exercice de test se termine, le procédé comprend en outre :
l'affichage, par le dispositif électronique, d'une troisième interface, dans lequel la troisième interface comprend l'âge de forme physique et un deuxième élément de commande, et le deuxième élément de commande est configuré pour déclencher le dispositif électronique afin de réestimer l'âge de forme physique ;
l'affichage, par le dispositif électronique, d'informations de guidage après que le dispositif électronique a détecté une deuxième opération effectuée par l'utilisateur sur le deuxième élément de commande, dans lequel les informations de guidage sont utilisées pour guider l'utilisateur pour achever un exercice de test pour tester la consommation maximale d'oxygène ; et
l'affichage, par le dispositif électronique, d'une quatrième interface après avoir détecté que l'exercice de test se termine, dans lequel la quatrième interface comprend un âge de forme physique mis à jour.

5. Procédé selon l'une quelconque des revendications 1 à 4, dans lequel le dispositif électronique comprend une relation de mise en correspondance entre un âge et une consommation d'oxygène ; et
le fait que le dispositif électronique obtient l'âge de forme physique en effectuant une mise en correspondance sur la base de la consommation maximale d'oxygène obtenue par test comprend :
l'interrogation, par le dispositif électronique, de la consommation maximale d'oxygène dans la relation de mise en correspondance, et la détermination d'un âge ayant une relation de mise en correspondance avec la consommation maximale d'oxygène en tant qu'âge de forme physique.

6. Procédé selon l'une quelconque des revendications 1 à 5, dans lequel le fait que le dispositif électronique teste la consommation maximale d'oxygène de l'utilisateur comprend :
le calcul, par le dispositif électronique, d'une distribution de probabilité de la consommation maximale d'oxygène sur la base d'une fréquence cardiaque d'exercice et d'une vitesse d'exercice de l'utilisateur pendant l'exercice de test ; et
la réalisation, par le dispositif électronique, d'une sommation pondérée sur une consommation d'oxygène et une probabilité correspondant à la consommation d'oxygène qui sont comprises dans la distribution de probabilité, pour obtenir la consommation maximale d'oxygène.

7. Procédé selon la revendication 6, dans lequel la correction (S1311 ; 1002, 1003), par le dispositif électronique, de l'âge de forme physique sur la base d'une durée d'exercice de l'utilisateur dans le premier intervalle de temps prédéfini, pour obtenir l'âge de forme physique corrigé comprend :
si l'âge de forme physique est supérieur à l'âge chronologique de l'utilisateur, la correction, par le dispositif électronique, de l'âge de forme physique en utilisant la formule suivante : $age 1 ' = age 0 + \left(age0-age1\right) \times p \times \left(1+n1\right)$
dans lequel age1' représente l'âge de forme physique corrigé, age0 représente l'âge chronologique, age1 représente l'âge de forme physique, p représente une probabilité cible correspondant à la consommation maximale d'oxygène dans la distribution de probabilité, et n1 représente un facteur de correction, dans lequel une durée d'exercice plus longue indique un facteur de correction plus petit, et n1 se situe dans une plage de [-1, 1].

8. Procédé selon l'une quelconque des revendications 1 à 7, dans lequel après que le dispositif électronique a obtenu l'âge de forme physique par mise en correspondance, le procédé comprend en outre :
la correction (S1315), par le dispositif électronique, de l'âge de forme physique sur la base d'un changement d'un état de santé de l'utilisateur dans un deuxième temps prédéfini, pour obtenir l'âge de forme physique corrigé, dans lequel
lorsque l'état de santé montre une tendance à l'amélioration, le dispositif électronique abaisse l'âge de forme physique ; ou lorsque l'état de santé montre une tendance à la détérioration, le dispositif électronique augmente l'âge de forme physique.

9. Procédé selon la revendication 8, dans lequel la correction (S1315), par le dispositif électronique, de l'âge de forme physique sur la base du changement de l'état de santé de l'utilisateur dans le deuxième temps prédéfini, pour obtenir l'âge de forme physique corrigé comprend au moins l'un des éléments suivants :
si une fréquence cardiaque au repos de l'utilisateur dans un deuxième intervalle de temps prédéfini montre une tendance à la diminution, l'abaissement, par le dispositif électronique, de l'âge de forme physique de n2 ans ; ou si une fréquence cardiaque au repos de l'utilisateur dans un deuxième intervalle de temps prédéfini montre une tendance à l'augmentation, l'augmentation, par le dispositif électronique, de l'âge de forme physique de n2 ans ;
si une fréquence cardiaque maximale de l'utilisateur dans un deuxième intervalle de temps prédéfini montre une tendance à l'augmentation, l'abaissement, par le dispositif électronique, de l'âge de forme physique de n3 ans ; ou si une fréquence cardiaque maximale de l'utilisateur dans un deuxième intervalle de temps prédéfini montre une tendance à la diminution, l'augmentation, par le dispositif électronique, de l'âge de forme physique de n3 ans ;
si une qualité de sommeil de l'utilisateur dans un deuxième intervalle de temps prédéfini montre une tendance à l'amélioration, l'abaissement, par le dispositif électronique, de l'âge de forme physique de n4 ans ; ou si une qualité de sommeil de l'utilisateur dans un deuxième intervalle de temps prédéfini montre une tendance à la détérioration, l'augmentation, par le dispositif électronique, de l'âge de forme physique de n4 ans ; ou
si un pourcentage de graisse corporelle de l'utilisateur dans un deuxième intervalle de temps prédéfini montre une tendance à la diminution, l'abaissement, par le dispositif électronique, de l'âge de forme physique de n5 ans ; ou si un pourcentage de graisse corporelle de l'utilisateur dans un deuxième intervalle de temps prédéfini montre une tendance à l'augmentation, l'augmentation, par le dispositif électronique, de l'âge de forme physique de n5 ans.

10. Procédé selon l'une quelconque des revendications 1 à 9, dans lequel après que le dispositif électronique a obtenu l'âge de forme physique par mise en correspondance, le procédé comprend en outre :
si une différence entre l'âge de forme physique et un quelconque âge de forme physique historique dans un troisième intervalle de temps prédéfini dépasse une différence prédéfinie, la correction, par le dispositif électronique, de l'âge de forme physique, pour obtenir un âge de forme physique corrigé, dans lequel une différence entre l'âge de forme physique corrigé et un quelconque âge de forme physique historique dans le troisième intervalle de temps prédéfini ne dépasse pas la différence prédéfinie.

11. Procédé selon l'une quelconque des revendications 6 à 10, dans lequel la correction de l'âge de forme physique comprend :
si l'âge de forme physique se situe dans une plage d'âge prédéfinie, la correction de l'âge de forme physique ; et
si l'âge de forme physique ne se situe pas dans la plage d'âge prédéfinie, l'ajustement, par le dispositif électronique, de l'âge de forme physique pour qu'il se situe dans la plage d'âge prédéfinie, et la correction de l'âge de forme physique.

12. Dispositif électronique, dans lequel le dispositif électronique comprend une mémoire (521) et un processeur (510), la mémoire (521) est couplée au processeur (510), la mémoire (521) stocke du code de programme informatique, le code de programme informatique comprend des instructions informatiques et, lorsque les instructions informatiques sont exécutées par le processeur (510), le dispositif électronique est rendu capable de mettre en œuvre le procédé selon l'une quelconque des revendications 1 à 11.

13. Support de stockage lisible par ordinateur, comprenant des instructions informatiques, dans lequel lorsque les instructions informatiques sont exécutées sur un dispositif électronique, le dispositif électronique est rendu capable de mettre en œuvre le procédé selon l'une quelconque des revendications 1 à 11.
